# EUROPÄISCHE PATENTSCHRIFT

(11) **EP 2 265 602 B1**
(45) Veröffentlichungstag und Bekanntmachung des Hinweises auf die Patenterteilung: **08.04.2015**
(21) Anmeldenummer: 09733899.0
(22) Anmeldetag: 11.04.2009
(51) Int. Cl.: C07D 401/14, C07D 403/14, C07D 413/14, A61K 31/506, A61K 31/5355, A61K 31/553, A61P 9/00, A61P 13/12, A61P 7/06

(54) **SUBSTITUIERTE DIHYDROPYRAZOLONE ALS HEMMER DER HIF-PROLYL-4-HYDROXYLASE**
SUBSTITUTED DIHYDROPYRAZOLONES AS INHIBITORS OF HIF-PROLYL-4-HYDROXYLASES
DIHYDROPYRAZOLONES SUBSTITUÉES UTILISÉES COMME INHIBITEURS DE LA HIF-PROLYL-4-HYDROXYLASE

(30) Priorität: 23.04.2008 DE 102008020113
(43) Veröffentlichungstag der Anmeldung: 29.12.2010
(73) Patentinhaber: Bayer Intellectual Property GmbH, 40789 Monheim (DE)
(72) Erfinder: JESKE, Mario, 42699 Solingen (DE); FLAMME, Ingo, 51580 Reichshof (DE); STOLL, Friederike, 40215 Düsseldorf (DE); BECK, Hartmut, 50733 Köln (DE); AKBABA, Metin, 40880 Ratingen (DE)
(74) Vertreter: BIP Patents
(86) Internationale Anmeldenummer: PCT/EP2009/002693
(87) Internationale Veröffentlichungsnummer: WO 2009/129945

(56) Entgegenhaltungen:
- WO-A1-2006/114213
- WO-A1-2008/067871

## Beschreibung

Die vorliegende Anmeldung betrifft neue, substituierte Dihydropyrazolon-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären und hämatologischen Erkrankungen, von Nierenerkrankungen sowie zur Förderung der Wundheilung.

Eine mangelhafte Versorgung des menschlichen Organismus oder seiner Teile mit Sauerstoff, die entweder durch ihre Dauer und/oder ihr Ausmaß eine regelrechte Funktion des Organismus oder seiner Teile beeinträchtigt oder ihre Funktion völlig zum Erliegen bringt, wird als Hypoxie bezeichnet. Eine Hypoxie kann verursacht werden durch eine Verminderung des verfügbaren Sauerstoffs in der Atemluft (z.B. bei Aufenthalten in großer Höhe), durch Störungen der äußeren Atmung (z.B. infolge gestörter Funktion der Lungen oder Verlegung der Atemwege), durch eine Verminderung des Herzzeitvolumens (z.B. bei einer Herzinsuffizienz, einer akuten Rechtsherzüberlastung bei Lungenembolie), durch eine zu geringe Sauerstoff-Transportkapazität des Blutes (z.B. infolge einer Blutarmut (Anämie) oder Intoxikation z.B. mit Kohlenmonoxid), örtlich begrenzt durch eine Minderdurchblutung infolge von Gefäßverschlüssen (Ischämie-Zustände typischerweise z.B. des Herzens, der unteren Extremitäten oder des Gehirns, diabetische Makro- und Mikroangiopathie) oder auch durch erhöhten Sauerstoffbedarf des Gewebes (z.B. infolge erhöhter Muskelarbeit oder lokaler Entzündungen) [Eder, Gedigk (Hrsg.), Allgemeine Pathologie und pathologische Anatomie, 33. Aufl., Springer Verlag, Berlin, 1990].

Der menschliche Organismus ist bedingt fähig, sich an Situationen verminderter Sauerstoffversorgung akut und chronisch anzupassen. Neben einer Sofortantwort, welche u.a. durch vegetativnervöse Kontrollmechanismen eine Erhöhung des Herzzeitvolumens und des Atemzeitvolumens sowie eine lokale Erweiterung von Blutgefäßen einschließt, zieht die Hypoxie eine Veränderung der Transkription zahlreicher Gene nach sich. Die Funktion der Genprodukte dient dabei der Kompensation des Sauerstoffmangels. So werden mehrere Enzyme der Glykolyse und der Glukosetransporter 1 verstärkt exprimiert, wodurch die anaerobe ATP-Gewinnung gesteigert und ein Überleben des Sauerstoffmangels ermöglicht wird [Schmidt, Thews (Hrsg.), Physiologie des Menschen, 27. Aufl., Springer Verlag, Berlin, 1997; Löffler, Petrides (Hrsg.), Biochemie und Pathobiochemie, 7. Aufl., Springer Verlag, Berlin, 2003].

Ferner führt Hypoxie zur verstärkten Expression des vaskulären Endothelzellwachstumsfaktors, VEGF, wodurch in hypoxischen Geweben die Neubildung von Blutgefäßen (Angiogenese) angeregt wird. Hierdurch wird langfristig die Durchblutung ischämischer Gewebe verbessert. Bei verschiedenen Herzkreislauferkrankungen und Gefäßverschluß-Erkrankungen erfolgt diese Gegen regulation offenbar nur sehr unzureichend [Übersicht bei: Simons und Ware, Therapeutic angiogenesis in cardiovascular disease, Nat. Rev. Drug. Discov. 2 (11), 863-71 (2003)].

Ferner wird bei systemischer Hypoxie das vorwiegend in den interstitiellen Fibroblasten der Nieren gebildete Peptidhormon Erythropoietin verstärkt exprimiert. Hierdurch wird die Bildung roter Blutzellen im Knochenmark angeregt und damit die Sauerstoff-Transportkapazität des Blutes gesteigert. Dieser Effekt wurde und wird von Leistungssportlern beim sogenannten Höhentraining genutzt. Eine Abnahme der Sauerstoff-Transportkapazität des Blutes z.B. infolge einer Blutungsanämie verursacht normalerweise eine Zunahme der Erythropoietin-Produktion in der Niere. Bei bestimmten Formen der Anämie kann dieser Regelmechanismus gestört oder sein Sollwert niedriger eingestellt sein. So wird z.B. bei Patienten, die unter einer Niereninsuffizienz leiden, Erythropoietin im Nierenparenchym zwar produziert, jedoch bezogen auf die Sauerstoff-Transportkapazität des Blutes in deutlich verminderten Mengen, was eine sogenannte renale Anämie zur Folge hat. Insbesondere die renale Anämie, aber auch Tumor- und HIV-Infektion-bedingte Anämien werden üblicherweise durch parenterale Verabfolgung von rekombinantem humanen Erythropoietin (rhEPO) behandelt. Derzeit existiert zu dieser kostspieligen Therapie keine alternative Therapie mit einem oral verfügbaren Arzneistoff [Übersichten bei: Eckardt, The potential of erythropoietin and related strategies to stimulate erythropoiesis, Curr. Opin. Investig. Drugs 2(8), 1081-5 (2001); Berns, Should the target hemoglobin flor patients with chronic kidney disease treated with erythropoietic replacement therapy be changed?, Semin. Dial. 18 (1), 22-9 (2005)]. Jüngere Untersuchungen belegen, dass Erythropoetin neben seiner die Erythropoese steigernden Wirkung auch eine hiervon unabhängige, schützende (anti-apoptotische) Wirkung auf hypoxische Gewebe, insbesondere das Herz und das Gehirn ausübt. Ferner mindert eine Therapie mit Erythropoetin neueren Studien zufolge an herzinsuffizienten Patienten den durchschnittlichen Morbiditäts-Schweregrad [Übersichten bei: Caiola und Cheng, Use of erythropoietin in heart failure management, Ann. Pharmacother. 38 (12), 2145-9 (2004); Katz, Mechanisms and treatment of anemia in chronic heart failure, Congest. Heart. Fail. 10 (5), 243-7 (2004)].

Den oben beschriebenen durch Hypoxie induzierten Genen ist gemeinsam, dass die Steigerung ihrer Expression unter Hypoxie durch den sogenannten Hypoxie-induzierbaren Transkriptionsfaktor (HIF) hervorgerufen wird. Bei HIF handelt es sich um einen heterodimeren Transkriptionsfaktor, der aus einer alpha- und einer beta-Untereinheit besteht. Es wurden drei HIF-alpha-Isoformen beschrieben, von denen HIF-1 alpha und HIF-2 alpha hoch homolog und von Bedeutung für die Hypoxie-induzierte Genexpression sind. Während die auch als ARNT (aryl hydrocarbon receptor nuclear translocator) bezeichnete beta-Untereinheit (von der 2 Isoformen beschrieben wurden) konstitutiv exprimiert ist, hängt die Expression der alpha-Untereinheit vom Sauerstoffgehalt in der Zelle ab. Unter Normoxie wird das HIF-alpha-Protein poly-ubiquitiniert und anschließend proteasomal degradiert. Unter Hypoxie ist diese Degradierung gehemmt, so dass HIF-alpha mit ARNT dimerisieren und seine Zielgene aktivieren kann. Das HIF-Dimer bindet hierbei an sogenannte Hypoxie-responsible Elemente (HRE) in den regulatorischen Sequenzen seiner Zielgene. Die HRE sind durch eine Konsensus-Sequenz definiert. Funktionelle HRE wurden in den regulatorischen Elementen zahlreicher Hypoxie-induzierter Gene nachgewiesen [Übersichten bei: Semenza, Hypoxia-inducible factor 1: oxygen homeostasis and disease pathophysiology, Trends Mol. Med. 7 (8), 345-50 (2001); Wenger und Gassmann, Oxygen(es) and the hypoxia-inducible facto-1, Biol. Chem. 378 (7), 609-16 (1997)].

Der molekulare Mechanismus, der dieser Regulation von HIF-alpha zugrunde liegt, wurde durch die Arbeiten mehrerer voneinander unabhängiger Forschergruppen aufgeklärt. Der Mechanismus ist Spezies-übergreifend konserviert: HIF-alpha wird durch eine als PHD oder EGLN bezeichnete Subklasse von Sauerstoff-abhängigen Prolyl-4-Hydroxylasen an zwei spezifischen Prolyl-Resten hydroxyliert (P402 und P564 der humanen HIF-1-alpha-Untereinheit). Bei den HIF-Prolyl-4-Hydroxylasen handelt es sich um Eisen-abhängige, 2-Oxoglutarat-umsetzende Dioxygenasen [Epstein et al., C. elegans EGL-9 and mammalian homologs define a family of dioxygenases that regulate HIF by prolyl hydroxylation, Cell 107 (1), 43-54 (2001); Bruick und McKnight, A conserved family of prolyl-4-hydroxylases that modify HIF, Science 294 (5545), 1337-40 (2001); Ivan et al., Biochemical purification and pharmacological inhibition of a mammalian prolyl hydroxylase acting on hypoxia-inducible factor, Proc. Natl. Acad. Sci. U.S.A. 99 (21), 13459-64 (2002)]. Die Enzyme wurden 2001 erstmals als Prolyl-Hydroxylasen annotiert [Aravind und Koonin, The DNA-repair protein AlkB, EGL-9, and leprecan define new families of 2-oxoglutarate- and iron-dependent dioxygenases, Genome Biol. 2 (3), research0007.1-0007.8, Epub 2001 Feb 19].

An die prolylhydroxylierte HIF-alpha-Untereinheit bindet das pVHL Tumor Suppressor Protein, welches zusammen mit Elongin B und C den sogenannten VBC-Komplex bildet, der die HIF-alpha-Untereinheit an eine E3 Ubiquitin-Ligase adaptiert. Da die Prolyl-4-Hydroxylierung der HIF-alpha-Untereinheit und ihre nachfolgende Degradierung in Abhängigkeit von der intrazellulären Konzentration des Sauerstoffs erfolgt, wurden die HIF-Prolyl-4-Hydroxylasen auch als zellulärer Sauerstoffsensor bezeichnet. Es wurden drei Isoformen dieser Enzyme identifiziert: EGLN1/PHD2, EGLN2/PHD1 und EGLN3/PHD3. Zwei dieser Enzyme (EGLN2/PHD1 und EGLN3/PHD3) werden selbst unter Hypoxie transkriptionell induziert und sind möglicherweise für die unter chronischer Hypoxie zu beobachtende Absenkung der HIF-alpha-Spiegel verantwortlich [Übersicht bei: Schofield und Ratcliffe, Oxygen sensing by HIF hydroxylases, Nat. Rev. Mol. Cell. Biol. 5 (5), 343-54 (2004)].

Eine selektive pharmakologische Hemmung der HIF-Prolyl-4-Hydroxylasen zieht die Steigerung der Genexpression HIF-abhängiger Zielgene nach sich und ist damit für die Therapie zahlreicher Krankheitsbilder von Nutzen. Insbesondere bei Erkrankungen des Herz-Kreislaufsystems ist von der Induktion neuer Blutgefäße sowie der Änderung der Stoffwechselsituation ischämischer Organe von aerober hin zu anaerober ATP-Gewinnung eine Besserung des Krankheitsverlaufs zu erwarten. Eine Verbesserung der Vaskularisierung chronischer Wunden fördert den Heilungsprozess, insbesondere bei schwer heilenden Ulcera cruris und anderen chronischen Hautwunden. Die Induktion von körpereigenem Erythropoietin bei bestimmten Krankheitsformen, insbesondere bei Patienten mit renaler Anämie, ist ebenfalls ein anzustrebendes therapeutisches Ziel.

Die bislang in der wissenschaftlichen Literatur beschriebenen HIF-Prolyl-4-Hydroxylase-Inhibitoren erfüllen nicht die an ein Arzneimittel zu stellenden Anforderungen. Es handelt sich hierbei entweder um kompetitive Oxoglutarat-Analoga (wie z.B. N-Oxalylglycin), die durch ihre sehr geringe Wirkstärke charakterisiert sind und daher in *in vivo*-Modellen bislang keine Wirkung im Sinne einer Induktion von HIF-Zielgenen gezeigt haben. Oder es handelt sich um Eisen-Komplexbildner (Chelatoren) wie Desferroxamin, die als unspezifische Hemmstoffe Eisen-haltiger Dioxygenasen wirken und, obwohl sie eine Induktion der Zielgene wie z.B. Erythropoetin *in vivo* herbeiführen, durch Komplexierung des verfügbaren Eisens offenbar der Erythropoese entgegenwirken.

2-Heteroaryl-4-aryl-1,2-dihydropyrazolone mit bakterizider und/oder fungizider Wirkung werden in EP 165 448 und EP 212 281 offenbart. Die Verwendung von 2-Heteroaryl-4-aryl-1,2-dihydro-pyrazolonen als Lipoxygenase-Inhibitoren zur Behandlung von Atemwegs-, Herz-Kreislauf- und Entzündungserkrankungen wird in EP 183 159 beansprucht. 2,4-Diphenyl-1,2-dihydropyrazolone mit herbizider Aktivität werden in DE 2 651 008 beschrieben. Über die Herstellung und pharmakologischen Eigenschaften bestimmter 2-Pyridyl-1,2-dihydropyrazolone wird in Helv. Chim. Acta 49 (1), 272-280 (1966) berichtet. In WO 96/12706, WO 00/51989 und WO 03/074550 werden Verbindungen mit Dihydropyrazolon-Partialstruktur zur Behandlung unterschiedlicher Erkrankungen beansprucht, und in WO 2006/101903 werden Hydroxy- oder Alkoxy-substituierte Bipyrazole zur Behandlung neuropsychiatrischer Erkrankungen offenbart. Weiterhin werden in WO 03/051833 und WO 2004/089303 Heteroaryl-substituierte Pyrazol-Derivate zur Behandlung von Schmerz und verschiedenen ZNS-Erkrankungen beschrieben. Zwischenzeitlich sind in WO 2006/114213 und WO 2008/067871 2,4-Dipyridyl-1,2-dihydropyrazolone als Inhibitoren von HIF-Prolyl-4-Hydroxylasen offenbart worden.

Über die Röntgenkristallstruktur der Verbindung 3-Methyl-1-(pyridin-2-yl)-4-(1-pyridin-2-yl-3-methyl-1*H*-pyrazol-5-yl)-2H-3-pyrazolin-5(1H)-on (andere Bezeichnung: 5,5'-Dimethyl-2,2'-di-pyridin-2-yl-1',2'-dihydro-2*H*,3'*H*-3,4'-bipyrazol-3'-on) wird in Acta Crystallogr., Section E: Structure Reports Online E57 (11), o1126-o1127 (2001) [Chem. Abstr. 2001:796190] berichtet. Die Synthese bestimmter 3',5-Dimethyl-2-phenyl-1'-(1,3-thiazol-2-yl)-1'*H*,2*H*-3,4'-bipyrazol-5'-ol-Derivate ist in Indian J. Heterocyclic Chem. 3 (1), 5-8 (1993) [Chem. Abstr. 1994:323362] beschrieben. Über Herstellung und Tautomerie einzelner 4-(Pyrazol-5-yl)-pyrazolin-5-on-Derivate wird in J. Heterocyclic Chem. 27 (4), 865-870 (1990) [Chem. Abstr. 1991:428557] berichtet. Eine therapeutische Anwendung ist für die in diesen Publikationen genannten Verbindungen bislang nicht beschrieben worden. In WO 2007/008541 wird die Verbindung 2-*tert*.-Butyl-1'-[4-(4-chlorphenyl)-1,3-thiazol-2-yl]-3',5-dimethyl-1'*H*,2*H*-3,4'-bipyrazol-5'-ol als ein Testbeispiel aufgeführt.

Aufgabe der vorliegenden Erfindung ist die Bereitstellung neuer Verbindungen, die für die Behandlung von Erkrankungen, insbesondere kardiovaskulärer und hämatologischer Erkrankungen, eingesetzt werden können.

Im Rahmen der vorliegenden Erfindung werden nunmehr Verbindungen beschrieben, die als spezifische Hemmstoffe der IIIF-Prolyl-4-Hydroxylasen wirken und aufgrund dieses spezifischen Wirkmechanismus *in vivo* nach parenteraler oder oraler Verabreichung die Induktion von HIF-Zielgenen, wie z.B. Erythropoetin, und der hierdurch verursachten biologischen Prozesse, wie z.B. Erythropoese, herbeiführen.

Gegenstand der Erfindung sind Verbindungen der Formel 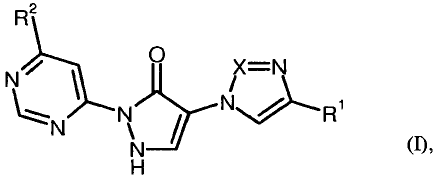 in welcher
- X: für N oder CH steht,
- R¹: für Wasserstoff oder Cyano steht,
- R²: für Piperazin-1-yl steht,
wobei Piperazin-1-yl substituiert ist mit einem Substituenten, wobei der Substituent ausgewählt wird aus der Gruppe bestehend aus C₃-C₆-Cycloalkyl,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich beiden von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl per se und "Alkyl" in Alkylamino stehen für einen linearen oder verzweigten Alkylrest mit 1 bis 3 Kohlenstoffatomen, beispielhaft und vorzugsweise für Methyl, Ethyl, n-Propyl, iso-Propyl.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, *N,N*-Dimethylamino, *N,N*-Diethylamino, *N*-Ethyl-*N*-methylamino, *N-*Methyl-*N*-n-propylamino und *N*-iso-Propyl-*N*-n-propylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.
Cycloalkyl steht für eine monocyclische Cycloalkylgruppe mit in der Regel 3 bis 6 Kohlenstoffatomen, beispielhaft und vorzugsweise für Cycloalkyl seien genannt Cyclopropyl, Cyclobutyl, Cyclopentyl und Cyclohexyl.
Über ein Stickstoffatom gebundener gesättigter 4- bis 7-gliedriger Heterocyclyl-Rest steht für einen monocyclischen, gesättigten, heterocyclischen Rest mit 4 bis 7 Ringatomen der ein Stickstoffatom enthält, über das er gebunden ist, und bis zu 2, vorzugsweise bis zu einem weiteren Heteroatom und/oder einer Heterogruppe aus der Reihe N, O, S, SO, SO₂, wobei ein Stickstoffatom auch ein N-Oxid bilden kann. Bevorzugt sind 4- bis 7-gliedrige, monocyclische gesättigte Heterocyclyl-Reste mit bis zu einem weiteren Heteroatom aus der Reihe O, N und S, beispielhaft und vorzugsweise für Azetidin-1-yl, Pyrrolin-1-yl, Piperidin-1-yl, Morpholin-4-yl, Thiomorpholin-4-yl, Piperazin-1-yl, 1,2-Oxazinan-2-yl, 1,4-Oxazepan-4-yl, 1,4-Thiazepan-4-yl.

Bevorzugt sind Verbindungen der Formel (I), in welcher
- X: für N oder CH steht,
- R¹: für Wasserstoff oder Cyano steht,
- R²: für Piperazin-1-yl steht,
wobei Piperazin-1-yl in 4-Position substituiert ist mit einem Substituenten, wobei der Substituent ausgewählt wird aus der Gruppe bestehend aus C₃-C₆-Cycloalkyl,
und ihre Salze, ihre Solvate und die Solvate ihrer Salze.

Bevorzugt sind auch Verbindungen der Formel (I), in welcher
- X: für N oder CH steht,
- R¹: für Wasserstoff steht,
- R²: für Piperazin-1-yl steht,
wobei Piperazin-1-yl in 4-Position substituiert ist mit einem Substituenten, wobei der Substituent ausgewählt wird aus der Gruppe bestehend aus C₃-C₆-Cycloalkyl,
und ihre Salze, ihre Solvate und die Solvate ihrer Salze.

Bevorzugt sind auch Verbindungen der Formel (I), in welcher X für N steht.

Bevorzugt sind auch Verbindungen der Formel (I), in welcher R¹ für Wasserstoff steht.

Bevorzugt sind auch Verbindungen der Formel (I), in welcher R¹ für Cyano steht.

Bevorzugt sind auch Verbindungen der Formel (I), in welcher R² für 4-Cyclobutyl-piperazin-1-yl steht.

Bevorzugt sind auch Verbindungen der Formel (I), in welcher
- X: für N oder CH steht,
- R¹: für Wasserstoff oder Cyano steht,
- R²: für 4-Cyclobutyl-piperazin-1-ylsteht,
und ihre Salze, ihre Solvate und die Solvate ihrer Salze.

Bevorzugt ist auch 2-[6-(4-Cyclobutylpiperazin-1-yl)pyrimidin-4-yl]-4-(1*H*-1,2,3-triazol-1-yl)-1,2-dihydro-3*H*-pyrazol-3-on mit der folgenden Formel 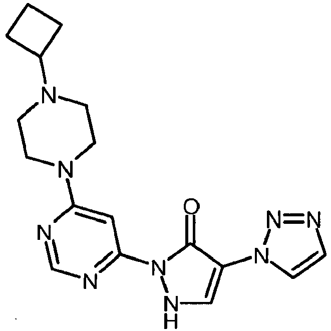 und ihre Salze, ihre Solvate und die Solvate ihrer Salze.

Bevorzugt ist auch 2-[6-(4-Cyclobutylpiperazin-1-yl)pyrimidin-4-yl]-4-(1*H*-1,2,3-triazol-1-yl)-1,2-dihydro-3*H*-pyrazol-3-on mit der folgenden Formel

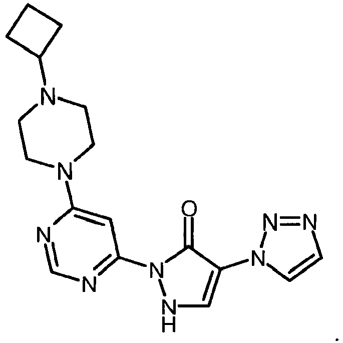

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

oder
- R²: für Piperazin-1-yl steht,
wobei Piperazin-1-yl in 4-Position substituiert ist mit einem Substituenten, wobei der Substituent ausgewählt wird aus der Gruppe bestehend aus C₃-C₆-Cycloalkyl,
oder
- R²: für Azetidin-1-yl steht,
wobei Azetidin-1-yl in 3-Position substituiert ist mit einem Substituenten, wobei der Substituent ausgewählt wird aus der Gruppe bestehend aus Hydroxycarbonyl, Methyl und Dimethylamino,
oder
- R²: für 1,2-Oxazinan-2-yl oder 1,4-Oxazepan-4-yl steht,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für über ein Stickstoffatom gebundenen gesättigten 4- bis 7-gliedrigen Heterocyclyl-Rest steht,
wobei der Heterocyclyl-Rest substituiert ist mit 1 bis 4 Substituenten Fluor,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für Azetidin-1-yl, Pyrrolin-1-yl oder Piperidin-1-yl steht,
wobei Azetidin-1-yl, Pyrrolin-1-yl und Piperidin-1-yl substituiert sind mit 1 bis 4 Substituenten Fluor,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für Azetidin-1-yl, Pyrrolin-1-yl oder Piperidin-1-yl steht,
wobei Azetidin-1-yl, Pyrrolin-1-yl und Piperidin-1-yl substituiert sind mit 2 Substituenten Fluor, wobei diese Substituenten an dasselbe Kohlenstoffatom gebunden sind,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für Piperazin-1-yl steht,
wobei Piperazin-1-yl substituiert ist mit einem Substituenten, wobei der Substituent ausgewählt wird aus der Gruppe bestehend aus C₃-C₆-Cycloalkyl,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für Piperazin-1-yl steht,
wobei Piperazin-1-yl in 4-Position substituiert ist mit einem Substituenten, wobei der Substituent ausgewählt wird aus der Gruppe bestehend aus C₃-C₆-Cycloalkyl,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für Azetidin-1-yl steht,
wobei Azetidin-1-yl substituiert ist mit einem Substituenten, wobei der Substituent ausgewählt wird aus der Gruppe bestehend aus Hydroxycarbonyl, C₁-C₃-Alkyl, C₁-C₃-Alkylamino und C₃-C₆-Cycloalkyl,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für Azetidin-1-yl steht,
wobei Azetidin-1-yl substituiert ist mit einem Substituenten, wobei der Substituent ausgewählt wird aus der Gruppe bestehend aus Hydroxycarbonyl, Methyl und Dimethylamino,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für Azetidin-1-yl steht,
wobei A,zetidin-1-yl in 3-Position substituiert ist mit einem Substituenten, wobei der Substituent ausgewählt wird aus der Gruppe bestehend aus Hydroxycarbonyl, Methyl und Dimethylamino,
und ihre Salze, ihre Solvate und die Solvate ihrer Salze.

Bevorzugt sind auch Verbindungen der Formel (I), in welcher
- X: für N oder CH steht,
- R¹: für Wasserstoff oder Cyano steht,
- R²: für 1,2-Oxazinan-2-yl oder 1,4-Oxazepan-4-yl steht.

Bevorzugt sind auch Verbindungen der Formel (I), in welcher X für N steht.

Bevorzugt sind auch Verbindungen der Formel (I), in welcher R¹ für Wasserstoff steht.

Bevorzugt sind auch Verbindungen der Formel (I), in welcher R¹ für Cyano steht.

Bevorzugt sind auch Verbindungen der Formel (I), in welcher R² für 4-Cyclobutyl-piperazin-1-yl steht.

Bevorzugt sind auch Verbindungen der Formel (I), in welcher
- X: für N oder CH steht,
- R¹: für Cyano steht,
- R²: für über ein Stickstoffatom gebundenen gesättigten 4- bis 7-gliedrigen Heterocyclyl-Rest steht,
wobei der Heterocyclyl-Rest substituiert ist mit einem Substituenten, wobei der Substituent ausgewählt wird aus der Gruppe bestehend aus Hydroxy, Hydroxycarbonyl, C₁-C₃-Alkyl, C₁-C₃-Alkylamino und C₃-C₆-Cycloalkyl,
oder
wobei der Heterocyclyl-Rest substituiert ist mit 1 bis 4 Substituenten Fluor,
und ihre Salze, ihre Solvate und die Solvate ihrer Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die erfindungsgemäßen 1,2-Dihydropyrazol-3-on-Derivate der Formel (I) können auch in der tautomeren 1H-Pyrazol-5-ol-Form (I') vorliegen (siehe nachfolgendes Schema 1); beide tautomeren Formen werden von der vorliegenden Erfindung ausdrücklich umfasst.

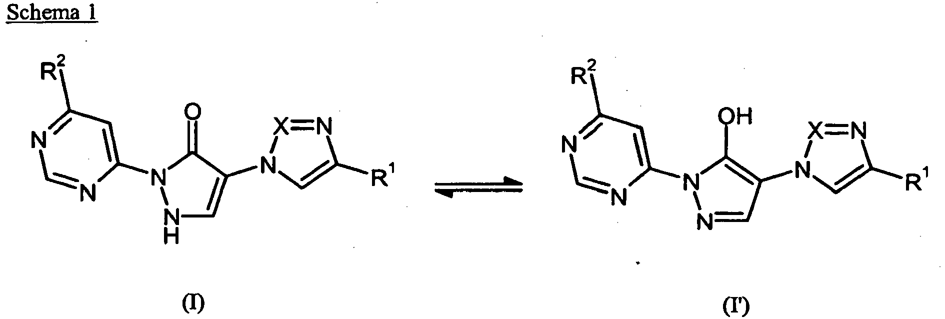

Gegenstand der Erfindung ist weiterhin ein Verfahren zur Herstellung der Verbindungen der Formel (I), oder ihrer Salze, ihrer Solvate oder der Solvate ihrer Salze, wobei nach Verfahren

### [A] Verbindungen der Formel

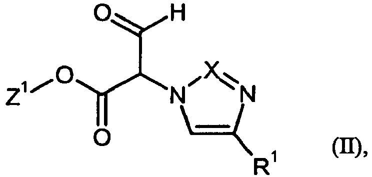

in welcher R¹ die oben angegebene Bedeutung hat, und
Z¹ für Methyl oder Ethyl steht,
in einem inerten Lösungsmittel gegebenenfalls in Gegenwart einer Säure mit einer Verbindung der Formel 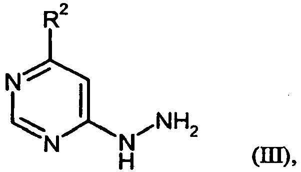 in welcher R² die oben angegebene Bedeutung hat,
zu Verbindungen der Formel 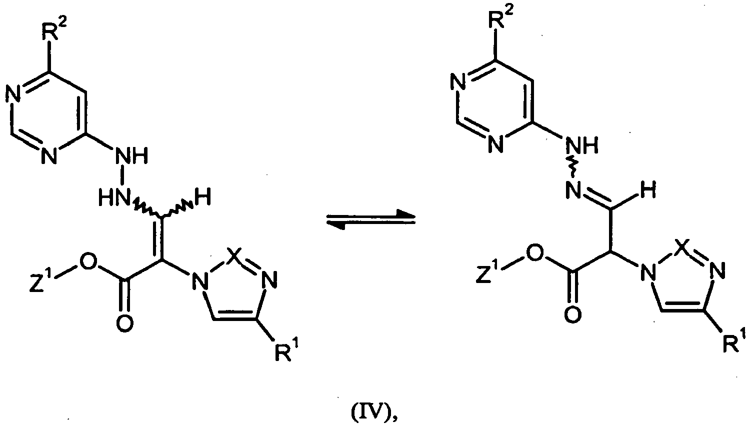 in welcher Z¹, R¹ und R² die oben angegebene Bedeutung haben,
umgesetzt werden, welche bereits unter diesen Reaktionsbedingungen oder in einem nachfolgenden Reaktionsschritt unter dem Einfluss einer Base zu den Verbindungen der Formel (I) cyclisieren,
und die Verbindungen der Formel (I) gegebenenfalls mit den entsprechenden (i) Lösungsmitteln und/oder (ii) Basen oder Säuren in ihre Salze, ihre Solvate oder die Solvate ihrer Salze überführt werden,
oder

### [B] Verbindungen der Formel

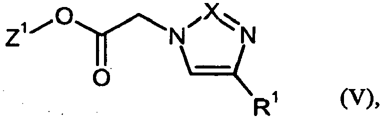

in welcher Z¹ und R¹ die oben angegebene Bedeutung haben,
mit einer Verbindung der Formel 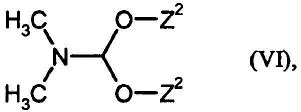 in welcher
- Z²: für Methyl oder Ethyl steht,
zu Verbindungen der Formel 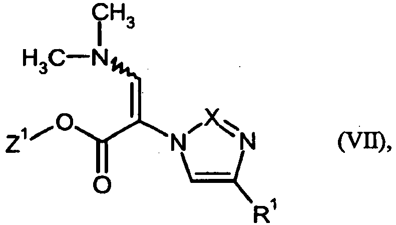 in welcher Z¹ und R¹ die oben angegebene Bedeutung haben,
kondensiert werden und anschließend in Gegenwart einer Säure mit einer Verbindung der Formel (III) zu Verbindungen der Formel (IV) umgesetzt werden, welche bereits unter diesen Reaktionsbedingungen oder in einem nachfolgenden Reaktionsschritt unter dem Einfluss einer Base zu den Verbindungen der Formel (I) cyclisieren,
und die Verbindungen der Formel (I) gegebenenfalls mit den entsprechenden (i) Lösungsmitteln und/oder (ii) Basen oder Säuren in ihre Salze, ihre Solvate und die Solvate ihrer Salze überführt werden,
oder

### [C] die Verbindung der Formel

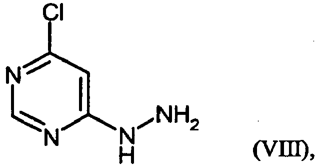

in Wasser als Lösungsmittel in einem Eintopfverfahren zunächst mit Verbindungen der Formel 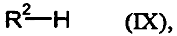 in welcher R² die oben angegebene Bedeutung hat,
und anschließend mit Verbindungen der Formel (VII) zu Verbindungen der Formel (I) umgesetzt werden,
und die Verbindungen der Formel (I) gegebenenfalls mit den entsprechenden (i) Lösungsmitteln und/oder (ii) Basen oder Säuren in ihre Salze, ihre Solvate und die Solvate ihrer Salze überführt werden.

Die freie Base der Salze kann durch Umsetzung der Salze der Verbindungen oder Solvate der Salze der Verbindungen mit einer Base gewonnen werden.

Als Basen eignen sich Alkalihydroxide wie beispielsweise Natrium- oder Kaliumhydroxid, Alkali- oder Erdalkalicarbonate wie Natrium-, Kalium-, Calcium- oder Cäsiumcarbonat, oder wässrige Ammoniklösung.

In einem alternativen Verfahren kann die freie Base der Salze zum Beispiel durch Chromatographie an einer Reversed Phase Säule mit einem Acetonitril-Wasser-Gradienten unter Zusatz einer Base erhalten werden, insbesondere durch Verwendung einer RP18 Phenomenex Luna C18(2) Säule und Diethylamin als Base.

Weiterer Gegenstand der Erfindung ist ein Verfahren zur Herstellung der Verbindungen der Formel (I) oder ihrer Solvate, bei dem Salze der Verbindungen oder Solvate der Salze der Verbindungen durch Umsetzung mit einer Base oder durch Chromatographie unter Zusatz einer Base in die Verbindungen überführt werden.

Weitere erfindungsgemäße Verbindungen können gegebenenfalls auch hergestellt werden durch Umwandlungen von fiunktionellen Gruppen einzelner Substituenten, insbesondere den unter R¹ und R² aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile oder elektrophile Substitution, Oxidation, Reduktion, Hydrierung, Übergangsmetall-katalysierte Kupplungsreaktionen, Alkylierung, Acylierung, Aminierung, Veresterung, Esterspaltung, Veretherung, Etherspaltung, Bildung von Carbonamiden, Sulfonamiden, Carbamaten und Harnstoffen, sowie die Einführung und Entfernung temporärer Schutzgruppen.

Als inerte Lösungsmittel für die Verfahrensschritte (II) + (III) → (IV), (VII) + (III) → (IV) und (IV) → (I) eignen sich insbesondere Ether wie Diethylether, Methyl-tert.-butylether, 1,2-Dimethoxyethan, Tetrahydrofuran und Dioxan, oder Alkohole wie Methanol, Ethanol, n-Propanol, isoPropanol, n-Butanol und tert.-Butanol, oder Wasser oder Gemische aus den Lösungsmitteln oder ein Gemisch aus einem Lösungsmittel mit Wasser. Bevorzugt werden Methanol, Ethanol, Tetrahydrofuran oder Wasser eingesetzt.

Der Verfahrensschritt (V) + (VI) → (VII) wird bevorzugt in Dimethylformamid als Lösungsmittel oder auch in Gegenwart eines Überschusses an (VI) ohne weiteres Lösungsmittel durchgeführt. Gegebenenfalls kann die Reaktion auch vorteilhaft unter Mikrowellen-Bestrahlung erfolgen. Die Umsetzung geschieht im Allgemeinen in einem Temperaturbereich von +20°C bis +150°C, bevorzugt bei +80°C bis +120°C [vgl. auch J.P. Bazureau et al., Synthesis 1998, 967; *ibid.* 2001 (4), 581].

Die Verfahrensschritte (II) + (III) → (IV) und (VII) + (III) → (IV) können gegebenenfalls vorteilhaft unter Zusatz einer Säure durchgeführt werden. Hierfür eignen sich übliche anorganische oder organische Säuren wie beispielsweise Chlorwasserstoff, Essigsäure, Trifluoressigsäure, Methansulfonsäure, p-Toluolsulfonsäure oder Campher-10-sulfonsäure. Bevorzugt werden Essigsäure oder insbesondere Trifluoressigsäure oder p-Toluolsulfonsäure verwendet.

Die Umsetzung (II) + (III) → (IV) erfolgt im Allgemeinen in einem Temperaturbereich von 0°C bis +100°C, bevorzugt bei +10°C bis +50°C. Die Reaktion (VII) + (III) → (IV) wird im Allgemeinen in einem Temperaturbereich von +20°C bis +120°C, bevorzugt bei +50°C bis +100°C durchgeführt.

Die Verfahrenssequenzen (II) + (III) → (IV) → (I) und (VII) + (III) → (IV) → (I) können unter zweistufiger Reaktionsführung oder auch als Eintopf-Reaktion, ohne Isolierung der Zwischenstufe (IV), durchgeführt werden. Für letztere Variante ist insbesondere die Umsetzung der Komponenten unter Mikrowellen-Bestrahlung geeignet; die Reaktion erfolgt hierbei im Allgemeinen in einem Temperaturbereich von +50°C bis +200°C, bevorzugt bei +100°C bis +180°C.

Teilweise tritt ein Ringschluss zu (I) auch bereits schon bei der Herstellung von (IV) ein; die Cyclisierung kann dann gegebenenfalls durch *in situ*-Behandlung des Reaktionsgemisches mit einer Base vervollständigt werden.

Als Base für einen solchen separaten Cyclisierungsschritt (IV) → (I) eignen sich übliche anorganische oder organische Basen. Hierzu gehören insbesondere Alkalihydroxide wie beispielsweise Natrium- oder Kaliumhydroxid, Alkali- oder Erdalkalicarbonate wie Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, oder Alkalihydride wie Natriumhydrid. Bevorzugt werden Natriummethanolat oder -ethanolat verwendet.

Die Basen-induzierte Umsetzung (IV) → (I) erfolgt im Allgemeinen in einem Temperaturbereich von 0°C bis +60°C, bevorzugt bei 0°C bis +30°C.

Die Umsetzung (VIII) + (IX) + (VII) → (I) erfolgt im Allgemeinen unter Verwendung von 1.1 bis 2.0 Äquivalenten (IX) bezogen auf 1 Äquivalent (VIII), gegebenenfalls in Gegenwart von 1.1 bis 2.0 Äquivalenten einer Base. Bevorzugt ist die Umsetzung mit 1.1 bis 1.5 Äquivalenten (IX).

Als Basen für die Umsetzung (VIII) + (IX) + (VII) → (I) eignen sich übliche anorganische oder organische Basen. Hierzu gehören insbesondere Alkalihydroxide wie beispielsweise Natrium- oder Kaliumhydroxid, oder Aminbasen wie beispielsweise *N*-Ethyl-*N*-(propan-2-yl)propan-2-amin. Bevorzugt ist *N*-Ethyl-*N*-(propan-2-yl)propan-2-amin.

Die Umsetzung (VIII) + (IX) + (VII) → (I) erfolgt im Allgemeinen in einem Temperaturbereich von +20°C bis +100°C, bevorzugt bei +70°C bis +100°C.

Alle Verfahrensschritte können bei normalem, erhöhtem oder bei erniedrigtem Druck durchgeführt werden (z.B. von 0.5 bis 5 bar). Im Allgemeinen arbeitet man bei Normaldruck.

Die Verbindungen der Formel (II) können nach literaturüblichen Methoden zur C-Acylierung von Carbonsäureestern aus Verbindungen der Formel (V) hergestellt werden. Die Verbindungen der Formeln (III), (V), (VI), (VIII) und (IX) sind kommerziell erhältlich, literaturbekannt oder können in Analogie zu in der Literatur beschriebenen Verfahren hergestellt werden.

Die Herstellung der erfindungsgemäßen Verbindungen kann durch das folgende Reaktionsschema 2 veranschaulicht werden:

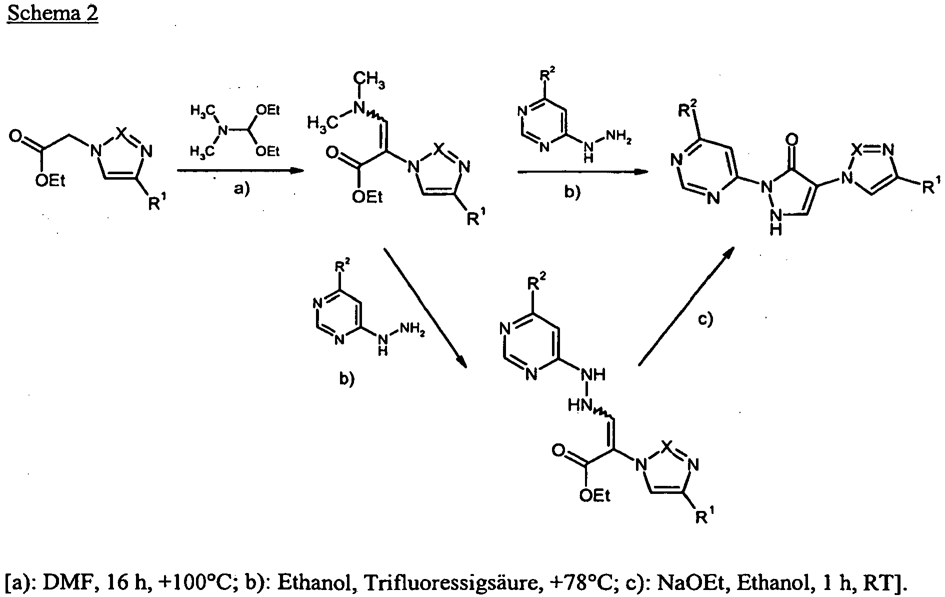

Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Die erfindungsgemäßen Verbindungen zeichnen sich als spezifische Hemmstoffe der HIF-Prolyl-4-Hydroxylasen aus.

Die erfindungsgemäßen Verbindungen können aufgrund ihrer pharmakologischen Eigenschaften zur Behandlung und/oder Prophylaxe von kardiovaskulären Krankheiten, insbesondere von Herzinsuffizienz, koronarer Herzkrankheit, Angina pectoris, Myokardinfarkt, Schlaganfall, Arteriosklerose, essentieller, pulmonaler und maligner Hypertonie sowie peripherer arterieller Verschlusskrankheit eingesetzt werden.

Die erfindungsgemäßen Verbindungen eignen sich weiterhin zur Behandlung und/oder Prophylaxe von Störungen der Blutbildung, wie z.B. idiopathischen Anämien, renaler Anämie und Anämien in Begleitung einer Tumor-Erkrankung (insbesondere einer Chemotherapie-induzierten Anämie), einer Infektion (insbesondere HIV-Infektion) oder einer anderen entzündlichen Erkrankung wie z.B. rheumatoider Arthritis. Die erfindungsgemäßen Verbindungen sind darüber hinaus geeignet zur unterstützenden Behandlung von Anämien infolge Blutverlust, Eisenmangel-Anämie, Vitaminmangel-Anämie (z.B. infolge Vitamin B12-Mangel oder infolge Folsäure-Mangel), hypoplastischer und aplastischer Anämie, hämolytischer Anämie oder zur unterstützenden Behandlung von Anämien in Folge von Eisenverwertungsstörungen (sideroachrestische Anämie) oder Anämien infolge anderer endokriner Störungen (z.B. Hypothyreose).

Die Verbindungen sind ferner geeignet zur Steigerung des Hämatokrits mit dem Ziel der Gewinnung von Blut zur Eigenblutspende vor Operationen.

Die erfindungsgemäßen Verbindungen können außerdem zur Behandlung und/oder Prophylaxe von operationsbedingten Ischämiezuständen und deren Folgeerscheinungen nach chirurgischen Eingriffen, insbesondere Eingriffen am Herzen unter Verwendung einer Herz-Lungenmaschine (z.B. Bypass-Operationen, Herzklappen-Implantationen), Eingriffen an den Halsschlagadern, Eingriffen an der Körperschlagader (Aorta) und Eingriffen mit instrumenteller Öffnung oder Durchdringung der Schädelkalotte verwendet werden. Die Verbindungen eignen sich ferner zur allgemeinen Behandlung und/oder Prophylaxe bei chirurgischen Eingriffen mit dem Ziel einer Beschleunigung der Wundheilung und Verkürzung der Rekonvaleszenzzeit.

Die Verbindungen sind darüber hinaus zur Behandlung und Prophylaxe von Folgeerscheinungen akuter und protrahierter Ischämiezustände des Gehirns geeignet (z.B. Schlaganfall, Geburtsasphyxie).

Die Verbindungen können ferner zur Behandlung und/oder Prophylaxe von Krebs und zur Behandlung und/oder Prophylaxe einer im Zuge der Behandlung von Krebs auftretenden Beeinträchtigung des Gesundheitszustandes insbesondere nach Therapie mit Zytostatika, Antibiotika und Bestrahlungen eingesetzt werden.

Die Verbindungen eignen sich ferner zur Behandlung und/oder Prophylaxe von Erkrankungen des rheumatischen Formenkreises und anderen den Autoimmunerkrankungen zuzurechnenden Krankheitsformen und insbesondere zur Behandlung und/oder Prophylaxe einer im Zuge der medikamentösen Behandlung derartiger Erkrankungen auftretenden Beeinträchtigung des Gesundheitszustandes.

Weiterhin können die erfindungsgemäßen Verbindungen eingesetzt werden zur Behandlung und/ oder Prophylaxe von Erkrankungen des Auges (z.B. Glaukom), des Gehirns (z.B. Morbus Parkinson, Morbus Alzheimer, Demenz, chronisches Schmerzempfinden), von chronischen Nierenerkrankungen, Niereninsuffizienz und akutem Nierenversagen sowie zur Förderung der Wundheilung.

Weiterhin eignen sich die Verbindungen zur Behandlung und/oder Prophylaxe einer insbesondere im höheren Lebensalter gehäuft auftretenden allgemeinen körperlichen Schwäche bis hin zur Kachexie.

Ferner eignen sich die Verbindungen zur Behandlung und/oder Prophylaxe sexueller Dysfunktion.

Außerdem sind die Verbindungen zur Behandlung und/oder Prophylaxe von Diabetes mellitus und seinen Folgeerkrankungen, wie z.B. diabetischer Makro- und Mikroangiopathie, diabetischer Nephropathie und Neuropathie, geeignet.

Weiterhin eignen sich die erfindungsgemäßen Verbindungen zur Behandlung und/oder Prophylaxe von fibrotischen Erkrankungen z.B. des Herzens, der Lunge und der Leber.

Insbesondere sind die erfindungsgemäßen Verbindungen auch zur Prophylaxe und Behandlung der Frühgeborenenretinopathie (Retinopathia praematurorum) geeignet.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt: ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika sowie Antibiotika.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Phosphodiesterase (PDE)-Inhibitor, wie beispielhaft und vorzugsweise Milrinone, Amrinone, Pimobendan, Cilostazol, Sildenafil, Vardenafil oder Tadalafil, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Tumor-Chemotherapeutikum verabreicht, beispielhaft und vorzugsweise aus der Gruppe der Platin-Komplexe, wie z.B. Cisplatin und Carboplatin, der Alkylantien, wie z.B. Cyclophosphamid und Chlorambucil, der Antimetabolite, wie z.B. 5-Fluoruracil und Methotrexat, der Topoisomerase-Hemmer, wie z.B. Etoposid und Camptothecin, der Antibiotika, wie z.B. Doxorubicin und Daunorubicin, oder der Kinase-Inhibitoren, wie z.B. Sorafenib und Sunitinib.

Bei einer bevorzugten Ausführungsform der Erfindung werden die erfindungsgemäßen Verbindungen in Kombination mit einem Antibiotikum verabreicht, beispielhaft und vorzugsweise aus der Gruppe der Penicilline, Cephalosporine oder Chinolone, wie z.B. Ciprofloxacin und Moxifloxacin.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- aq.: wässrige Lösung
- cat.: katalytisch
- d: Tag(e)
- DCI: direkte chemische Ionisation (bei MS)
- DMF: Dimethylformamid
- DMSO: Dimethylsulfoxid
- d. Th.: der Theorie (bei Ausbeute)
- EI: Elektronenstoß-Ionisation (bei MS)
- ESI: Elektrospray-Ionisation (bei MS)
- Et: Ethyl
- GC-MS: Gaschromatographie-gekoppelte Massenspektroskopie
- h: Stunde(n)
- HPLC: Hochdruck-, Hochleistungsflüssigchromatographie
- konz.: konzentriert
- LC-MS: Flüssigchromatographie-gekoppelte Massenspektroskopie
- Meth.: Methode
- min.: Minute(n)
- MS: Massenspektroskopie
- NMR: Kernresonanzspektroskopie
- Rₜ: Retentionszeit (bei HPLC)
- RT: Raumtemperatur
- TFA: Trifluoressigsäure
- THF: Tetrahydrofuran

### LC-MS-, GC-MS- und HPLC-Methoden:

Methode 1 (LC-MS): Instrument: Micromass Platform LCZ mit HPLC Agilent Serie 1100; Säule: Thermo Hypersil GOLD 3µ, 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

Methode 2 (LC-MS): Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

Methode 3 (LC-MS): Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

Methode 4 (LC-MS): Instrument: Micromass Quattro Micro MS mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

Methode 5 (LC-MS): Instrument: Micromass QuattroPremier mit Waters UPLC Acquity; Säule: Thermo Hypersil GOLD 1.9µ 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

Methode 6 (HPLC): Instrument: HP 1100 mit DAD-Detektion; Säule: Kromasil 100 RP-18, 60 mm x 2.1 mm, 3.5 µm; Eluent A: 5 ml Perchlorsäure (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

Methode 7 (GC-MS): Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

Methode 8 (präparative HPLC): Säule: Kromasil 100 C18 5 µm, 250 mm x 20 mm; Eluent A: Milli-Q-Wasser, Eluent B: wässrige 0.1%-ige Trifluoressigsäure, Eluent C: Acetonitril; Gradient: 0.0 min 76% A, 5% B, 19% C → 15 min 4% A, 95% B, 1% C → 15.1 min 76% A, 5% B, 19% C → 20 min 76% A, 5% B, 19% C; Ofen: 40°C; Fluss: 25 ml/min; UV-Detektion: 210 nm.

Methode 9 (präparative HPLC): Säule: Sunfire C18 5 µm, 19 mm x 150 mm; Eluent A: wässrige 0.2%-ige Trifluoressigsäure, Eluent B: Acetonitril; Gradient: 0.0 min 95% A → 8 min 50% A → 8.01 min 95% A → 12 min 95% A; RT; Fluss: 25 ml/min; UV-Detektion: 210 nm.

Methode 10 (präparative HPLC): Säule: Sunfire C18 5 µm, 19 mm x 150 mm; Eluent A: wässrige 0.2%-ige Trifluoressigsäure, Eluent B: Acetonitril; 0 min 90% A → 13 min 90% A; Ofen: 40°C; Fluss: 25 ml/min; UV-Detektion: 210 nm.

Methode 11 (präparative HPLC): Säule: XBridge C18 5 µm, 19 mm x 150 mm; Eluent A: wässrige 0.2%-ige Ameisensäure, Eluent B: Acetonitril; 0 min 75% A → 6 min 75% A; RT; Fluss: 25 ml/min; UV-Detektion: 210 nm.

Methode 12 (präparative HPLC): Säule: XBridge C18 5 µm, 19 mm x 150 mm; Eluent A: wässrige 0.2%-ige Ameisensäure, Eluent B: Acetonitril; 0 min 93% A → 4 min 93% A; RT; Fluss: 25 ml/min; UV-Detektion: 210 nm.

Methode 13 (präparative HPLC): Säule: XBridge C18 5 µm, 19 mm x 150 mm; Eluent A: wässrige 0.2%-ige Trifluoressigsäure, Eluent B: Acetonitril; 0 min 90% A → 12 min 90% A; Ofen: 40°C; Fluss: 25 ml/min; UV-Detektion: 210 nm.

### Ausgangsverbindungen

### Beispiel 1A

### (4-Cyano-1H-imidazol-1-yl)essigsäureethylester

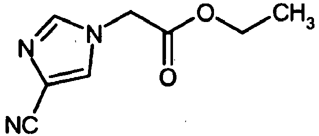

3.3 g (35.3 mmol) 1*H*-Imidazol-4-carbonitril [Matthews et al., J. Org. Chem. 1986, 51, 3228-3231] werden in 13.2 ml (11.5 g, 35.3 mmol) 2.1%-iger Natriumethylat-Lösung in Ethanol vorgelegt und mit 4.3 ml (6.5 g, 38.9 mmol) Bromessigsäureethylester versetzt. Die Reaktionsmischung wird 16 h bei RT gerührt. Zur Aufarbeitung wird der ausgefallene Feststoff abfiltriert, der Filterrückstand mit Ethanol gewaschen und das Filtrat im Vakuum aufkonzentriert. Der Rückstand wird mit Diisopropylether versetzt, nochmals filtriert, das Filtrat wieder am Rotationsverdampfer eingeengt und der Rückstand im Vakuum getrocknet. Ausbeute: 3.8 g (60% d. Th.)
LC-MS (Methode 1): Rₜ = 1.17 min; MS (ESIpos): m/z = 180 [M+H]⁺;
¹H-NMR (400 MHz, DMSO-d₆): δ = 8.12 (s, 1H), 7.88 (s, 1H), 5.06 (s, 2H), 4.18 (q, 2H), 1.22 (t, 3H).

### Beispiel 2A

### 2-(1H-1,2,3-Triazol-1-yl)essigsäureethylester

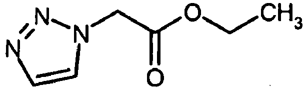

129.2 g (5.6 mol) Natrium werden langsam zu 4.0 Liter Ethanol gegeben. Anschließend werden 400.0 g (5.6 mol) 1,2,3-1*H*-Triazol zugesetzt sowie 623 ml (938.2 g, 5.6 mol) Bromessigsäureethylester bei 20-25°C Innentemperatur zugetropft. Es wird 48 h bei RT gerührt. Der ausgefallene Feststoff wird abfiltriert, das Ethanol im Vakuum entfernt und erneut filtriert. Der Rückstand wird in Essigsäureethylester aufgenommen, filtriert, erneut im Vakuum eingeengt und destillativ an einer 30 cm-Kolonne aufgereinigt. Das Produkt wird bei einer Badtemperatur von 140°C, einer Kopftemperatur von 60-115°C und einem Druck von 1 mbar erhalten. Ausbeute: 440.0 g (50% d. Th.)
HPLC (Methode 6): Rₜ = 1.58 min;
LC-MS (Methode 1): Rₜ = 0.71 min; MS (ESIpos): m/z = 156 [M+H]⁺.

### Beispiel 3A

### 1H-Imidazol-1-ylessigsäureethylester

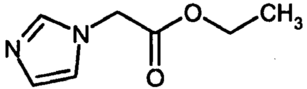

118.2 g (5.1 mol) Natrium werden langsam zu 2.5 Liter Ethanol gegeben. Anschließend werden 350.0 g (5.1 mol) Imidazol zugesetzt sowie 570 ml (858.6 g, 5.1 mol) Bromessigsäureethylester bei 20-25°C Innentemperatur zugetropft. Es wird 24 h bei RT gerührt. Der ausgefallene Feststoff wird abfiltriert, das Ethanol im Vakuum entfernt und erneut filtriert. Der Rückstand wird säulenchromatographisch an Kieselgel aufgereinigt (Laufmittel: Essigsäureethylester). Ausbeute: 639.0 g (81% d. Th.)
GC-MS (Methode 7): Rₜ = 4.55 min; MS (ESIpos): m/z = 155 [M+H]⁺.

### Beispiel 4A

### (4-Cyano-1H-1,2,3-triazol-1-yl)essigsäureethylester

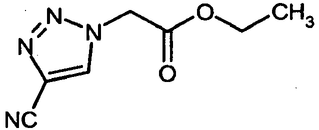

4.1 g (31.9 mmol) Azidoessigsäureethylester und 2.8 g (31.9 mmol) 2-Chloracrylsäurenitril werden in 32 ml Wasser 16 h bei einer Badtemperatur von 80°C gerührt. Nach Abkühlen auf RT wird die Lösung mit 1 N Salzsäure sauer gestellt und mit Essigsäureethylester extrahiert. Die organische Phase wird über Natriumsulfat getrocknet, filtriert und im Vakuum eingeengt. Der Rückstand wird mit 50 ml Ethanol und 10 Tropfen konzentrierter Schwefelsäure versetzt und 16 h unter Rückfluss gerührt. Zur Aufarbeitung wird die Reaktionsmischung im Vakuum aufkonzentriert, der Rückstand mit Essigsäureethylester versetzt, die Suspension mit halbkonzentrierter Natriumhydrogencarbonat-Lösung gewaschen und die organische Phase über Natriumsulfat getrocknet. Das Lösungsmittel wird am Rotationsverdampfer vollständig entfernt und der Feststoff im Vakuum getrocknet. Ausbeute: 1.5 g (25% d. Th.)
LC-MS (Methode 3): Rₜ = 0.96 min; MS (ESIpos): m/z = 181 [M+H]⁺;
¹H-NMR (400 MHz, DMSO-d₆): δ = 9.06 (s, 1H), 5.57 (s, 2H), 4.19 (q, 2H), 1.22 (t, 3H).

### Beispiel 5A

### 3-(N,N-Dimethylamino)-2-(1H-imidazol-1-yl)acrylsäureethylester

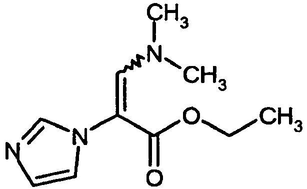

38.0 g (244.9 mmol) der Verbindung aus Beispiel 3A werden in 126 ml (108.1 g, 734.7 mmol) *N,N*-Dimethylformamid-diethylacetal 16 h bei 90°C Badtemperatur gerührt. Nach dem Abkühlen engt man im Vakuum ein, verrührt mit Diisopropylether, filtriert den Feststoff ab und wäscht diesen abschließend mit Diisopropylether. Ausbeute: 49.0 g (95% d. Th.)
LC-MS (Methode 2): Rₜ = 2.42 min; MS (ESIpos): m/z = 211 [M+H]⁺;
¹H-NMR (400 MHz, DMSO-d₆): δ = 7.52 (s, 1H), 7.49 (s, 1H), 7.05 (s, 1H), 6.91 (s, 1H), 4.02 (q, 2H), 2.63 (br. s, 6H), 1.12 (t, 3H).

### Beispiel 6A

### 3-(N,N-Dimethylamino)-2-(4-cyano-1H-imidazol-1-yl)acrylsäureethylester

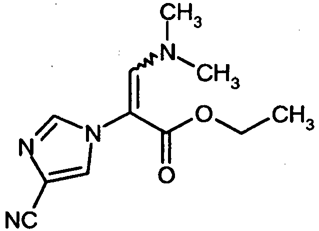

3.8 g (21.4 mmol) der Verbindung aus Beispiel 1A und 7.4 ml (6.3 g, 42.8 mmol) *N,N*-Dimethylformamid-diethylacetal werden bei 100°C Badtemperatur 16 h lang gerührt. Zur Aufarbeitung wird die abgekühlte Reaktionslösung am Rotationsverdampfer eingeengt und der Rückstand im Vakuum getrocknet. Ausbeute: 5.0 g (73% Reinheit, 73% d. Th.)
LC-MS (Methode 1): R, = 2.69 min; MS (ESIpos): m/z = 235 [M+H]⁺;
¹H-NMR (400 MHz, DMSO-d₆): δ = 8.13 (s, 1H), 7.85 (s, 1H), 7.58 (s, 1H), 4.03 (q, 2H), 2.69 (br. s, 6H), 1.12 (t, 3H).

### Beispiel 7A

### 3-(Dimethylamino)-2-(4-cyano-1H-1,2,3-triazol-1-yl)acrylsäureethylester

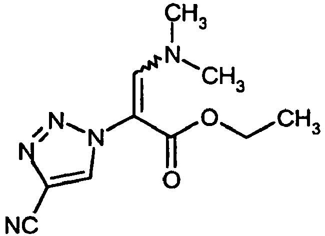

1.3 g (7.5 mmol) der Verbindung aus Beispiel 4A und 1.4 ml (1.2 g, 8.2 mmol) *N,N*-Dimethylformamid-diethylacetal werden bei 100°C Badtemperatur 16 h lang gerührt. Zur Aufarbeitung wird die abgekühlte Reaktionslösung am Rotationsverdampfer eingeengt und der Rückstand im Vakuum getrocknet. Ausbeute: 1.5 g (86% d. Th.)
LC-MS (Methode 4): Rₜ = 1.55 min; MS (ESIpos): m/z = 236 [M+H]⁺;
¹H-NMR (400 MHz, DMSO-d₆): δ = 9.14 (s, 1H), 7.75 (s, 1H), 4.04 (q, 2H), 3.15 (br. s, 3H), 2.18 (br. s, 3H), 1.13 (t, 3h).

### Beispiel 8A

### 3-(Dimethylamino)-2-(1H-1,2,3-triazol-1-yl)acrylsäureethylester

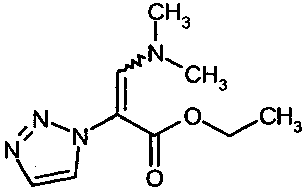

20.0 g (128.9 mmol) der Verbindung aus Beispiel 2A werden mit 44.2 ml (38.0 g, 257.8 mmol) N,N-Dimethylformamid-diethylacetal versetzt und 16 h bei 100°C gerührt. Nach Abkühlen auf RT wird das Reaktionsgemisch im Vakuum eingeengt. Der Rückstand wird in Diethylether verrührt, filtriert und mit Diethylether gewaschen. Ausbeute: 18.0 g (67% d. Th.)
LC-MS (Methode 4): R, = 1.20 min; MS (ESIpos): m/z = 211 [M+H]⁺.
¹H-NMR (400 MHz, DMSO-d₆): δ = 8.10 (d, 1H), 7.78 (d, 1H), 7.65 (s, 1H), 4.03 (q, 2H), 3.06 (br. s, 3H), 2.10 (br. s, 3H), 1.12 (t, 3H).

### Beispiel 9A

### 4-(4-Cyclobutylpiperazin-1-yl)-6-hydrazinopyrimidin

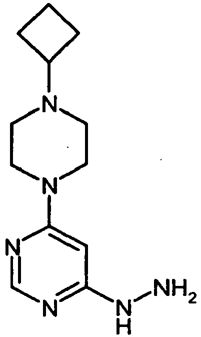

### Stufe a): 4-Chlor-6-(4-cyclobutylpiperazin-1-yl)pyrimidin

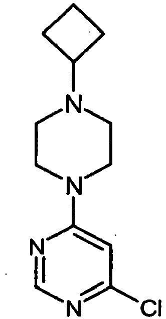

1.8 g (8.4 mmol) 1-Cyclobutylpiperazindihydrochlorid (Zaragoza et al., J. Med. Chem. 2004, 47, 2833) werden in 18 ml Wasser vorgelegt und mit 2.9 ml (2.1 g, 16.9 mmol) *N*-Ethyl-*N*-(propan-2-yl)propan-2-amin versetzt. Man rührt 30 min. bei RT und gibt 1.3 g (8.4 mmol) 4,6-Dichlorpyrimidin zu. Das Reaktionsgemisch wird 1 h bei 115°C gerührt, auf RT abgekühlt, mit 25 ml Essigsäureethylester versetzt und mit 25 ml einer gesättigten, wässrigen Natriumhydrogencarbonat-Lösung extrahiert. Die organische Phase wird abgetrennt, über Natriumsulfat getrocknet, filtriert und im Vakuum eingeengt. Das Rohprodukt wird säulenchromatographisch an Kieselgel aufgereinigt (Laufmittel: Dichlormethan/Methanol 100/3). Ausbeute: 1.9 g (89% d. Th.)
HPLC (Methode 6): Rₜ = 2.79 min; MS (DCI): m/z = 254 [M+H]⁺;
¹H-NMR (400 MHz, DMSO-d₆): δ = 8.32 (s, 1H), 6.95 (s, 1H), 3.65-3.58 (m, 4H), 2.70 (quintett, 1H), 2.27 (t, 4H), 2.00-1.93 (m, 2H), 1.87-1.75 (m, 2H), 1.67-1.55 (m, 2H).

### Stufe b): 4-(4-Cyclobutylpiperazin-1-yl)-6-hydrazinopyrimidin

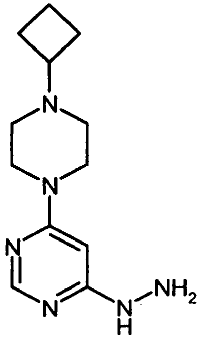

In eine Lösung von 1.9 g (7.5 mmol) 4-Chlor-6-(4-cyclobutylpiperazin-1-yl)pyrimidin in 28 ml Ethanol werden unter Rühren 4.4 ml (4.5 g, 89.7 mmol) Hydrazinhydrat bei RT zugetropft. Die Reaktionslösung wird 16 h bei 80°C nachgerührt. Zur Aufarbeitung wird im Vakuum eingeengt, der Rückstand in Diethylether mehrmals verrührt, der ausgefallene Feststoff abfiltriert und im Vakuum getrocknet. Anschließend wird der Rückstand säulenchromatographisch an Kieselgel aufgereinigt (Laufmittel: Dichlormethan/Methanol 10/2). Ausbeute: 1.5 g (80% d. Th.)
LC-MS (Methode 6): Rₜ = 1.36 min; MS (ESIpos): m/z = 249 [M+H]⁺;
¹H-NMR (400 MHz, DMSO-d₆): δ = 7.92 (s, 1H), 7.63 (s, 1H), 5.90 (NH), 4.09 (s, NH2), 3.45 (t, 4H), 2.69 (quintett, 1H), 2.26 (t, 4H), 2.00-1.93 (m, 2H), 1.82-1.75 (m, 2H), 1.67-1.60 (m, 2H).

### Beispiel 10A

### 1-(6-Hydrazinylpyrimidin-4-yl)azetidin-3-ol

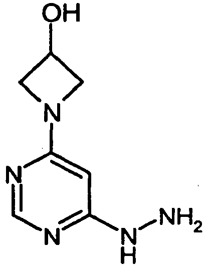

### Stufe a): 1-(6-Chlorpyrimidin-4-yl)azetidin-3-ol

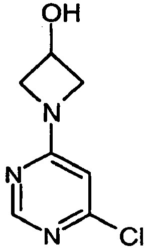

7.3 g (48.7 mmol) 4,6-Dichlorpyrimidin werden in 140 ml Wasser suspendiert und mit 47 ml 1 N Natronlauge versetzt. 5.3 g (48.7 mmol) 3-Hydroxyazetidin werden zugegeben, und das Reaktionsgemisch wird 3 d bei 90°C gerührt. Nach Abkühlen auf RT wird das Reaktionsgemisch im Vakuum eingeengt und ohne Reinigung weiter umgesetzt.
LC-MS (Methode 5): Rₜ = 0.36 min; MS (ESIpos): m/z = 1.87 [M+H]⁺.

### Stufe b): 1-(6-Hydrazinylpyrimidin-4-yl)azetidin-3-ol

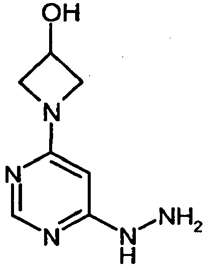

In eine Lösung von 10.4 g (55.8 mmol) 1-(6-Chlorpyrimidin-4-yl)azetidin-3-ol in 100 ml Ethanol werden unter Rühren 27.2 ml (27.9 g, 279.1 mmol) Hydrazinhydrat bei RT zugetropft. Die Reaktionslösung wird 16 h bei 80°C nachgerührt. Zur Aufarbeitung wird im Vakuum eingeengt, der Niederschlag abfiltriert und zweimal mit je 10 ml Ethanol gewaschen. Ausbeute: 2.0 g (19% d. Th.)
LC-MS (Methode 1): Rₜ = 2.06 min; MS (ESIpos): m/z = 194 [M+H]⁺.

### Beispiel 11A

### 4-Chlor-6-hydrazinopyrimidin

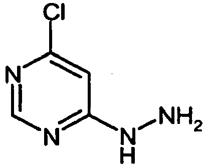

In eine Lösung von 20.0 g (134.3 mmol) 4,6-Dichlorpyrimidin in 300 ml Ethanol werden unter Rühren 11.8 ml (12.1 g, 241.6 mmol) Hydrazinhydrat bei RT zugetropft. Tritt eine Trübung der Lösung während der Zudosierung des Hydrazinhydrats auf, gibt man weiteres Ethanol (ca. 400 ml) hinzu. Die Reaktionslösung wird 12 h bei RT nachgerührt. Zur Aufarbeitung wird der ausgefallene Feststoff abfiltriert, der Filterrückstand zweimal mit je 150 ml Wasser und zweimal mit je 100 ml Diethylether gewaschen und das Produkt im Vakuum getrocknet. Aus der eingeengten Mutterlauge wird eine weitere kristalline Produktfraktion erhalten. Ausbeute: 16.8 g (87% d. Th.)
LC-MS (Methode 1): Rₜ = 1.17 min; MS (ESIpos): m/z = 145 [M+H]⁺;
¹H-NMR (400 MHz, DMSO-d₆): δ = 8.81 (s, 1H), 8.17 (br. s, 1H), 6.75 (s, 1H), 4.48 (br. s, 2H).

### Beispiel 12A

### 2-(6-Chlorpyrimidin-4-yl)-4-(1H-1,2,3-triazol-1-yl)-1,2-dihydro-3H-pyrazol-3-on-Hydrochlorid

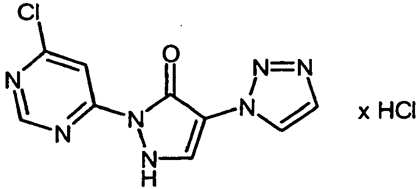

10.0 g (47.7 mmol) der Verbindung aus Beispiel 8A und 8.3 g (57.1 mmol) der Verbindung aus Beispiel 11A werden in 100 ml Ethanol vorgelegt und mit 1.5 ml (2.2g, 19.0 mmol) Trifluoressigsäure versetzt. Es wird 12 h unter Rückfluss gerührt. Anschließend gibt man der abgekühlten Reaktionsmischung eine 4 M Lösung von Chlorwasserstoff in Dioxan im Überschuss zu, rührt ca. 1 h aus, filtriert die ausgefallenen Kristalle ab und wäscht den Filterrückstand mit Dioxan und Ethanol. Das so erhaltene Zwischenprodukt wird in 150 ml Ethanol gelöst, mit 50 ml einer 25%-igen methanolischen Natriummethylat-Lösung versetzt und 2 h bei RT gerührt. Anschließend wird die Reaktionsmischung mit 1 N Salzsäure auf pH = 5 gestellt, weitere 2 h bei RT ausgerührt, der Feststoff abfiltriert, der Filterrückstand mit Ethanol gewaschen und das Produkt im Vakuum getrocknet. Ausbeute: 7.0 g (49% d. Th.)
LC-MS (Methode 5): Rₜ = 1.20 min; MS (ESIpos): m/z = 264 [M+H]⁺.

### Beispiel 13A

### 2-(6-Chlorpyrimidin-4-yl)-4-(1H-imidazol-1-yl)-1,2-dihydro-3H-pyrazol-3-on-Hydrochlorid

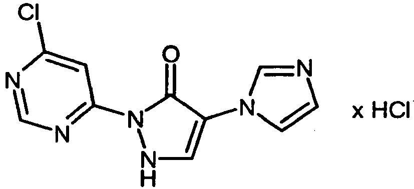

10.0 g (47.8 mmol) der Verbindung aus Beispiel 5A und 8.3 g (57.3 mmol) der Verbindung aus Beispiel 11A werden in 100 ml Ethanol vorgelegt und mit 1.5 ml (2.2 g, 19.0 mmol) Trifluoressigsäure versetzt. Es wird 12 h unter Rückfluss gerührt. Man filtriert die ausgefallenen Kristalle ab, wäscht den Filterrückstand mit Ethanol nach und trocknet das Zwischenprodukt über Nacht im Vakuum. Dieses wird dann in 20 ml Methanol suspendiert, mit 100 ml einer 4 M Lösung von Chlorwasserstoff in Dioxan versetzt und 1 h bei RT nachgerührt. Der Feststoff wird abfiltriert, der Filterrückstand mit Dioxan, Essigsäureethylester und Diisopropylether gewaschen und das Produkt im Vakuum getrocknet. Ausbeute: 4.6 g (32% d.Th.)
HPLC (Methode 6): Rₜ = 2.81 min; MS (ESIpos): m/z = 263 [M+H]⁺;
¹H-NMR (400 MHz, DMSO-d₆): δ = 9.46 (s, 1H), 8.96 (s, 1H), 8.56 (s, 1H), 8.51 (d, 1H), 8.07-8.04 (m, 1H), 7.85-7.82 (m, 1H).

### Beispiel 14A

### 4-(6-Hydrazinylpyrimidin-4-yl)-1,4-oxazepan

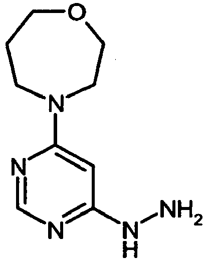

### Stufe a): 4-(6-Chlorpyrimidin-4-yl)-1,4-oxazepan

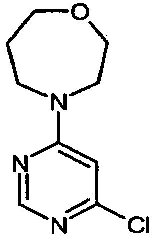

Ein Gemisch aus 3.0 g (20.1 mmol) 4,6-Dichlorpyrimidin, 2.8 g (20.1 mmol) 1,4-Oxazepanhydrochlorid und 6.4 g (60.4 mmol) Natriumcarbonat in 45 ml Wasser wird 16 h unter Rückfluss gerührt. Nach Abkühlen auf RT wird das Reaktionsgemisch mit Essigsäureethylester extrahiert. Die organische Phase wird mit gesättigter Natriumchlorid-Lösung gewaschen, über Natriumsulfat getrocknet und filtriert. Das Filtrat wird im Vakuum bis zur Trockne eingeengt. Das Produkt wird als Öl erhalten. Ausbeute: 3.9 g (86% d. Th.)
LC-MS (Methode 4): Rₜ = 1.32 min; MS (ESIpos): m/z = 214 [M+H]⁺;
¹H-NMR (400 MHz, DMSO-d₆): δ = 8.33 (s, 1H), 6.86 (s, 1H), 3.99-3.52 (m, 8H), 1.84 (m, 2H).

### Stufe b): 4-(6-Hydrazinylpyrimidin-4-yl)-1,4-oxazepan

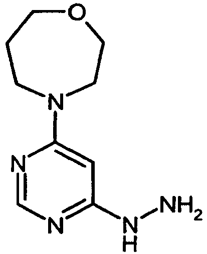

In eine Lösung von 3.9 g (18.0 mmol) 4-(6-Chlorpyrimidin-4-yl)-1,4-oxazepan in 25 ml Ethanol werden unter Rühren 8.8 ml (9.0 g, 180.2 mmol) Hydrazinhydrat bei RT zugetropft. Nach 16 h Rühren bei 80°C wird die Reaktionslösung im Vakuum eingeengt. Der Rückstand wird in kaltem Ethanol verrührt, der ausgefallene Feststoff abfiltriert und der Filterrückstand mit 25 ml Diethylether gewaschen. Das Produkt wird im Vakuum getrocknet. Ausbeute: 1.4 g (36% d. Th.)
HPLC (Methode 11): Rₜ = 2.48 min; MS (ESIpos): m/z = 210 [M+H]⁺;
¹H-NMR (400 MHz, DMSO-d₆): δ = 7.91 (s, 1H), 7.56 (br. s, 1H), 5.81 (s, 1H), 4.12 (br. s, 2H), 3.75-3.55 (m, 8H), 1.85 (quintett, 2H).

### Ausführungsbeispiele

### Beispiel 1

### 2-[6-(4-Cyclobutylpiperazin-1-yl)pyrimidin-4-yl]-4-(1H-imidazol-1-yl)-1,2-dihydro-3H-pyrazol-3-on

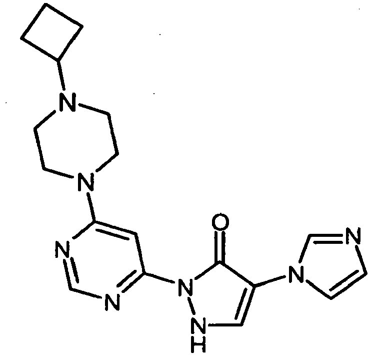

Ein Gemisch aus 211 mg (1.0 mmol) der Verbindung aus Beispiel 5A und 250 mg (1.0 mmol) der Verbindung aus Beispiel 9A in 4 ml Essigsäureethylester wird mit 16 µl (23 mg, 0.2 mmol) Trifluoressigsäure versetzt und 20 h bei 100°C gerührt. Das Reaktionsgemisch wird im Vakuum eingeengt, erneut mit der gleichen Menge Essigsäureethylester und Trifluoressigsäure versetzt und erneut 20 h bei 100°C gerührt. Das Reaktionsgemisch wird auf RT abgekühlt, der ausgefallene Feststoff abfiltriert und mit Diethylether gewaschen. Der Rückstand wird säulenchromatographisch an Kieselgel vorgereinigt (Laufmittel: Dichlormethan/Methanol/ Ammoniak 10/2/0.2) und mittels präparativer HPLC (RP18-Säule; Laufmittel: Acetonitril/Wasser-Gradient) aufgereinigt. Ausbeute: 137 mg (36% d. Th.)
HPLC (Methode 6): Rₜ = 2.73 min; MS (ESIpos): m/z = 367 [M+H]⁺;
¹H-NMR (400 MHz, DMSO-d₆): δ = 8.40 (s, 1H), 8.12 (s, 1H), 7.90 (s, 1H), 7.74 (s, 1H), 7.50 (s, 1H), 7.07 (s, 1H), 3.65-3.58 (m, 4H), 2.77 (quintett, 1H), 2.38-2.35 (m, 4H), 2.01-1.96 (m, 2H), 1.89-1.79 (m, 2H), 1.67-1.62 (m, 2H).

### Beispiel 2

### 2-[6-(4-Cyclobutylpiperazin-1-yl)pyrimidin-4-yl]-4-(1H-1,2,3-triazol-1-yl)-1,2-dihydro-3H-pyrazol-3-on

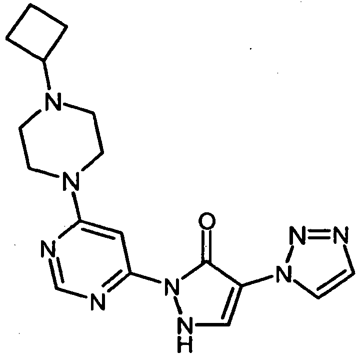

Ein Gemisch aus 211 mg (1.0 mmol) der Verbindung aus Beispiel 8A und 250 mg (1.0 mmol) der Verbindung aus Beispiel 9A in 4 ml Essigsäureethylester wird mit 16 µl (23 mg, 0.2 mmol) Trifluoressigsäure versetzt und 20 h bei 100°C gerührt. Das Reaktionsgemisch wird im Vakuum eingeengt, erneut mit der gleichen Menge Essigsäureethylester und Trifluoressigsäure versetzt und 3 d bei 100°C gerührt. Das Reaktionsgemisch wird auf RT abgekühlt, der ausgefallene Feststoff abfiltriert und mit Diethylether gewaschen. Der Rückstand wird in 2 ml Wasser suspendiert, durch Zugabe von wässriger 1 N Natronlauge gelöst (pH = 9-10) und mittels präparativer HPLC (RP18-Säule; Laufmittel: Acetonitril/Wasser-Gradient) aufgereinigt. Ausbeute: 195 mg (51% d. Th.)
HPLC (Methode 6): Rₜ = 2.90 min; MS (ESIpos): m/z = 368 [M+H]⁺;
¹H-NMR (400 MHz, DMSO-d₆): δ = 8.41 (s, 1H), 8.40 (s, 1H), 7.88 (s, 1H), 7.82 (s, 1H), 7.75 (s, 1H), 3.70-3.65 (m, 4H), 3.03-2.97 (m, 1H), 2.60-2.57 (m, 4H), 2.06-2.02 (m, 2H), 1.98-1.90 (m, 2H), 1.70-1.64 (m, 2H).

### Beispiel 3

### 1-{2-[6-(4-Cyclobutylpiperazin-1-yl)pyrimidin-4-yl]-3-oxo-2,3-dihydro-1H-pyrazol-4-yl}-1H-imidazol-4-carbonitril

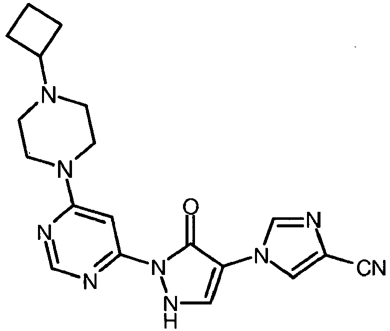

Ein Gemisch aus 236 mg (1.0 mmol) der Verbindung aus Beispiel 6A und 250 mg (1.0 mmol) der Verbindung aus Beispiel 9A in 4 ml Essigsäureethylester wird mit 16 µl (23 mg, 0.2 mmol) Trifluoressigsäure versetzt und 20 h bei 100°C gerührt. Das Reaktionsgemisch wird im Vakuum eingeengt, erneut mit der gleichen Menge Essigsäureethylester und Trifluoressigsäure versetzt und 3 d bei 100°C gerührt. Das Reaktionsgemisch wird auf RT abgekühlt, der ausgefallene Feststoff abfiltriert und mit Diethylether gewaschen. Der Rückstand wird in 2 ml Wasser suspendiert, durch Zugabe von wässriger 1 N Natronlauge gelöst (pH = 9-10) und mittels präparativer HPLC (RP18-Säule; Laufmittel: Acetonitril/Wasser-Gradient) aufgereinigt. Ausbeute: 219 mg (56% d. Th.)
HPLC (Methode 6): Rₜ = 3.10 min; MS (ESIpos): m/z = 392 [M+H]⁺;
¹H-NMR (400 MHz, DMSO-d₆): δ = 8.38 (d, 1H), 8.37 (d, 1H), 8.17 (d, 1H), 7.82 (s, 1H), 7.80 (s, 1H), 3.68-3.62 (m, 4H), 3.38-3.30 (m, 4H), 2.96-2.91 (m, 1H), 2.06-2.00 (m, 2H), 1.95-1.85 (m, 2H), 1.70-1.63 (m, 2H).

### Vergleichsbeispiele 4-29:

### Beispiel 4

### 1-{2-[6-(3-Hydroxyazetidin-1-yl)pyrimidin-4-yl]-3-oxo-2,3-dihydro-1H-pyrazol-4-yl}-1H-imidazol-4-carbonitril

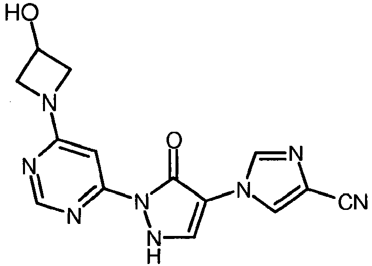

Ein Gemisch aus 700 mg (3.0 mmol) der Verbindung aus Beispiel 6A und 541 mg (3.0 mmol) der Verbindung aus Beispiel 10A in 10 ml Essigsäureethylester wird mit 46 µl (68 mg, 0.6 mmol) Trifluoressigsäure versetzt und 10 h bei 100°C gerührt. Das Reaktionsgemisch wird im Vakuum eingeengt, in 5 ml Ethanol aufgenommen, und der Niederschlag wird abfiltriert. Der Feststoff wird in 10 ml Wasser suspendiert und bis zur Lösung mit 1 N Natronlauge (pH = 9) versetzt. Anschließend wird mit 1 N Salzsäure pH = 7 eingestellt, auf ca. 5 ml Volumen eingeengt und der entstandene Niederschlag abfiltriert. Der Rückstand wird mit Wasser und Diisopropylether gewaschen und mittels präparativer HPLC (Methode 8) chromatographiert. Anschließend wird der Feststoff in 10 ml Wasser suspendiert und bis zur Lösung mit 1 N Natronlauge (pH = 9) versetzt. Anschließend wird mit 1 N Salzsäure pH = 7 eingestellt, auf ca. 2.5 ml Volumen eingeengt und der entstandene Niederschlag abfiltriert. Das Filtrat wird auf ca. 2 ml eingeengt und erneut filtriert. Beide Rückstände werden vereinigt, mit Wasser und Essigsäureethylester gewaschen und im Vakuum getrocknet. Ausbeute: 78 mg (8% d. Th.)
HPLC (Methode 6): Rₜ = 2.90 min; MS (ESIpos): m/z = 325 [M+H]⁺;
¹H-NMR (400 MHz, DMSO-d₆): δ = 8.35 (s, 1H), 8.29 (s, 1H), 8.17 (s, 1H), 7.66 (s, 1H), 7.34 (s, 1H), 5.80-5.75 (m, 1H), 4.63-4.58 (m, 1H), 4.24-4.20 (m, 2H), 3.75-3.73 (m, 2H).

### Beispiel 5

### 1-{6-[4-(1H-Imidazol-1-yl)-5-oxo-2,5-dihydro-1H-pyrazol-1-yl]pyrimidin-4-yl}azetidin-3-carbonsäure

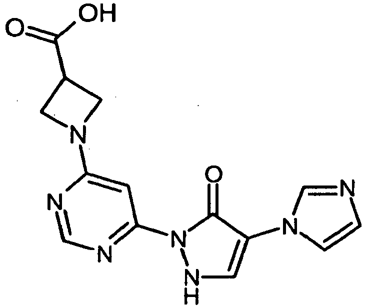

46 mg (0.3 mmol) Azetidin-3-carbonsäurehydrochlorid werden in einem Gemisch aus 1 ml Wasser und 0.3 ml Ethanol vorgelegt. 100 mg (0.3 mmol) der Verbindung aus Beispiel 13A werden zugegeben und 1 h bei 100°C gerührt. Anschließend wird das Reaktionsgemisch mit wässriger 1 N Natronlauge pH = 7 eingestellt und 16 h bei 100°C gerührt. Es wird erneut mit wässriger 1 N Natronlauge pH = 7 einstellt und 1 h bei 150°C in einer *single mode*-Mikrowelle (Emrys Optimizer) umgesetzt. Das Reaktionsgemisch wird im Vakuum eingeengt und mittels präparativer HPLC (RP18-Säule; Laufmittel: Acetonitril/Wasser-Gradient) aufgereinigt. Ausbeute: 23 mg (21% d. Th.)
LC-MS (Methode 8): Rₜ = 0.86 min; MS (ESIpos): m/z = 328 [M+H]⁺;
¹H-NMR (400 MHz, DMSO-d₆): δ = 8.41 (s, 1H), 7.79 (s, 1H), 7.47 (s, 1H), 7.36 (s, 1H), 7.31 (s, 1H), 6.89 (s, 1H), 4.09 (t, 2H), 3.99 (t, 2H).

### Beispiel 6

### 1-{6-[5-Oxo-4-(1H-1,2,3-triazol-1-yl)-2,5-dihydro-1H-pyrazol-1-yl]pyrimidin-4-yl}azetidin-3-carbonsäure

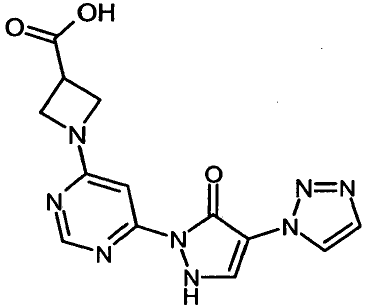

55 mg (0.4 mmol) Azetidin-3-carbonsäurehydrochlorid werden in 2 ml Wasser vorgelegt. 100 mg (0.3 mmol) der Verbindung aus Beispiel 12A werden zugegeben und mit wässriger 1 N Natronlauge pH = 7 eingestellt. Es wird 1 h bei 150°C in einer *single mode*-Mikrowelle (Emrys Optimizer) umgesetzt. Das Reaktionsgemisch wird im Vakuum eingeengt und mittels präparativer HPLC (RP18-Säule; Laufmittel: Acetonitril/Wasser-Gradient) aufgereinigt. Ausbeute: 15 mg (13% d. Th.)
HPLC (Methode 6): Rₜ = 2.81 min; MS (ESIpos): m/z = 329 [M+H]⁺;
¹H-NMR (400 MHz, DMSO-d₆): δ = 8.42 (s, 1H), 8.26 (s, 1 H), 7.70 (s, 1H), 7.67 (s, 1H), 7.34 (s, 1H), 4.05-3.96 (m, 2H und 2H), 3.13-3.07 (m, 1H).

### Beispiel 7

### 4-(1H-Imidazol-1-yl)-2-[6-(3-methylazetidin-1-yl)pyrimidin-4-yl]-1,2-dihydro-3H-pyrazol-3-on

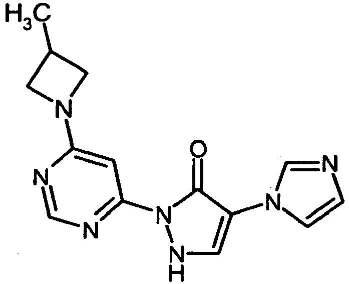

43 mg (0.4 mmol) 3-Methylazetidinhydrochlorid, 100 mg (0.3 mmol) der Verbindung aus Beispiel 13A und 174 µl (130 mg, 1.0 mmol) *N*-Ethyl-*N*-(propan-2-yl)propan-2-amin werden in 2 ml Tetrahydrofuran suspendiert und 4.5 h bei 120°C in einer *single mode-*Mikrowelle (Emrys Optimizer) umgesetzt. Das Reaktionsgemisch wird im Vakuum eingeengt, in Wasser unter Zusatz von wässriger 1 N Natronlauge (pH = 9-10) aufgenommen und mittels präparativer HPLC (RP18-Säule; Laufmittel:: Acetonitril/Wasser-Gradient) aufgereinigt. Ausbeute: 30 mg (30% d. Th.)
HPLC (Methode 6): Rₜ = 3.07 min; MS (ESIpos): m/z = 298 [M+H]⁺;
¹H-NMR (400 MHz, DMSO-d₆): δ = 8.25 (s, 1H), 7.81 (s, 1H), 7.46 (s, 1H), 7.39 (s, 1H), 7.31 (s, 1H), 6.88 (s, 1H), 4.10 (t, 2H), 3.54 (dd, 2H), 2.86-2.75 (m, 1H), 1.25 (d, 3H).

### Beispiel 8

### 2-[6-(3-Methylazetidin-1-yl)pyrimidin-4-yl]-4-(1H-1,2,3-triazol-1-yl)-1,2-dihydro-3H-pyrazol-3-on

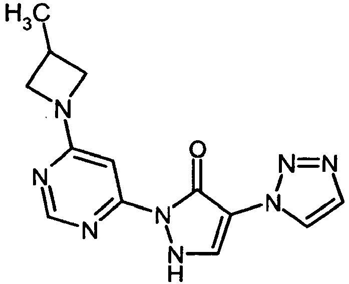

43 mg (0.4 mmol) 3-Methylazetidinhydrochlorid, 100 mg (0.3 mmol) der Verbindung aus Beispiel 12A und 174 µl (130 mg, 1.0 mmol) *N*-Ethyl-N-(propan-2-yl)propan-2-amin werden in 2 ml Tetrahydrofuran suspendiert und 1.5 h bei 120°C in einer *single mode-*Mikrowelle (Emrys Optimizer) umgesetzt. Das Reaktionsgemisch wird im Vakuum eingeengt, in Wasser unter Zusatz von wässriger 1 N Natronlauge (pH = 9-10) aufgenommen und mittels präparativer HPLC (RP18 Säule; Laufmittel: Acetonitril/Wasser-Gradient) aufgereinigt. Ausbeute: 25 mg (25% d. Th.)
HPLC (Methode 6): Rₜ = 3.00 min; MS (ESIpos): m/z = 299 [M+H]⁺;
¹H-NMR (400 MHz, DMSO-d₆): δ = 8.41 (s, 1H), 8.27 (s, 1H), 7.70 (s, 1H), 7.67 (s, 1H), 7.41 (s, 1H), 4.11 (t, 2H), 3.56 (dd, 2H), 2.86-2.78 (m, 1H), 1.25 (d, 3H).

### Beispiel 9

### 2-{6-[3-(Dimethylamino)azetidin-1-yl]pyrimidin-4-yl}-4-(1H-1,2,3-triazol-1-yl)-1,2-dihydro-3H-pyrazol-3-on Dihydrochlorid

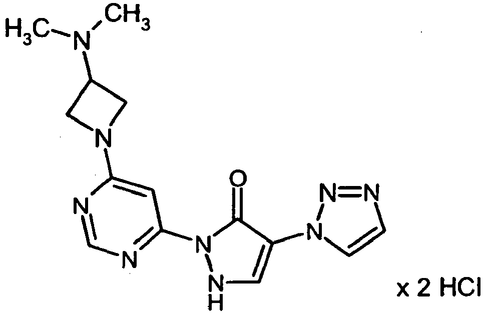

271 mg (1.5 mmol) *N*,*N*-Dimethylazetidin-3-amindihydrochlorid, 400 mg (1.5 mmol) der Verbindung aus Beispiel 12A und 847 mg (6.1 mmol) Kaliumcarbonat werden in 8 ml N,N-Dimethylformamid suspendiert und 16 h bei 100°C gerührt. Das Reaktionsgemisch wird im Vakuum eingeengt und mittels präparativer HPLC (RP18-Säule; Laufmittel: Acetonitril/Wasser-Gradient unter Zusatz von 0.1%-iger Trifluoressigsäure) gereinigt. Weitere Reinigung erfolgt mittels präparativer HPLC (RP18-Säule; Laufmittel: Acetonitril/Wasser-Gradient unter Zusatz von 0.1%-iger Ameisensäure). Die produkthaltigen Fraktionen werden mit 2 ml 1 N Salzsäure versetzt und 1 h bei RT gerührt. Der Feststoff wird abfiltriert und im Hochvakuum getrocknet. Ausbeute: 62 mg (17% d. Th.)
LC-MS (Methode 5): Rₜ = 0.19 min; MS (ESIpos): m/z = 328 [M+H]⁺;
¹H-NMR (400 MHz, DMSO-d₆): δ = 8.42 (s, 1H), 8.28 (s, 1H), 7.69 (s, 1H), 7.66 (s, 1H), 7.52 (s, 1H), 4.02 (t, 2H), 3.76 (dd, 2H), 3.24-3.18 (m, 1H), 2.12 (s, 6H).

### Beispiel 10

### 2-[6-(4,4-Difluorpiperidin-1-yl)pyrimidin-4-yl]-4-(1H-imidazol-1-yl)-1,2-dihydro-3H-pyrazol-3-on

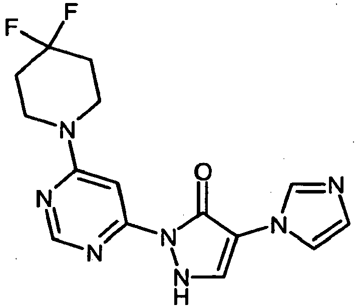

100 mg (0.3 mmol) der Verbindung aus Beispiel 13A, 63 mg (0.4 mmol) 4,4-Difluorpiperidinhydrochlorid und 116 µl (86 mg, 0.7 mmol) *N*-Ethyl-*N*-(propan-2-yl)propan-2-amin werden in 2 ml Tetrahydrofuran vorgelegt und 2.5 h bei 120°C in einer *single mode*-Mikrowelle (Emrys Optimizer) umgesetzt. Nach Einengen im Vakuum wird der Rückstand in Acetonitril/Wasser aufgenommen und mittels präparativer HPLC (RP18-Säule; Laufmittel: Acetonitril/Wasser-Gradient) gereinigt. Ausbeute: 82 mg (71% d. Th.)
HPLC (Methode 6): Rₜ = 3.37 min; MS (ESIpos): m/z = 348 [M+H]⁺;
¹H-NMR (400 MHz, DMSO-d₆): δ = 8.49 (s, 1H), 8.38 (s, 1H), 8.15 (s, 1H), 7.76 (s, 1H), 7.64 (s, 1H), 7.22 (s, 1H), 3.80 (t, 4H), 2.06 (heptett, 4H).

### Beispiel 11

### 2-[6-(4,4-Difluorpiperidin-1-yl)pyrimidin-4-yl]-4-(1H-1,2,3-triazol-1-yl)-1,2-dihydro-3H-pyrazol-3-on

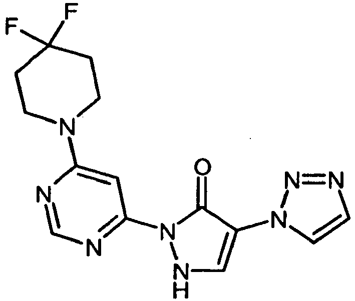

250 mg (0.8 mmol) der Verbindung aus Beispiel 12A, 158 mg (1.0 mmol) 4,4-Difluorpiperidinhydrochlorid und 435 µl (323 mg, 2.5 mmol) *N*-Ethyl-*N*-(propan-2-yl)propan-2-amin werden in 5 ml Tetrahydrofuran vorgelegt und 30 min bei 120°C in einer *single mode*-Mikrowelle (Emrys Optimizer) umgesetzt. Nach einer Vorreinigung mittels präparativer HPLC (RP18-Säule; Laufmittel: Acetonitril/Wasser-Gradient) wird das Produkt zusätzlich säulenchromatographisch an Kieselgel (Laufmittel: Dichlormethan/Methanol, 10/1) gereinigt. Ausbeute: 29 mg (10% d. Th.)
HPLC (Methode 6): Rₜ = 3.49 min; MS (ESIpos): m/z = 349 [M+H]⁺;
¹H-NMR (400 MHz, DMSO-d₆): δ = 8.56 (s, 1H), 8.39 (s, 1H), 8.30 (s, 1H), 7.87 (s, 1H), 7.57 (s, 1H), 3.91-3.81 (m, 4H), 2.09 (heptett, 4H).

### Beispiel 12

### 1-{2-[6-(4,4-Difluorpiperidin-1-yl)pyrimidin-4-yl]-3-oxo-2,3-dihydro-1H-pyrazol-4-yl}-1H-imidazol-4-carbonitril

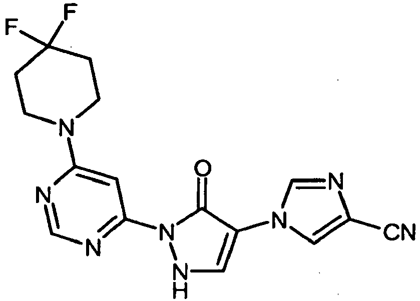

Ein Gemisch aus 200 mg (1.4 mmol) der Verbindung aus Beispiel 11A, 262 mg (1.7 mmol) 4,4-Difluorpiperidinhydrochlorid und 289 µl (215 mg, 1.7 mmol) *N*-Ethyl-*N*-(propan-2-yl)propan-2-amin in 3 ml Wasser wird 16 h bei 100°C gerührt. Nach Zugabe von 53 µl (79 mg, 0.7 mmol) Trifluoressigsäure und 324 mg (1.4 mmol) der Verbindung aus Beispiel 6A wird das Reaktionsgemisch 16 h bei 100°C gerührt. Der ausgefallene Feststoff wird abfiltriert und zunächst mit Wasser, dann mit Diethylether gewaschen. Das Produkt wird im Vakuum getrocknet.
Ausbeute: 111 mg (21% d. Th.)
LC-MS (Methode 4): Rₜ = 1.69 min; MS (ESIpos): m/z = 373 [M+H]⁺;
¹H-NMR (400 MHz, DMSO-d₆): δ = 8.55 (s, 1H), 8.44 (d, 1H), 8.33 (s, 1H), 8.22 (d, 1H), 7.60 (br. s, 1H), 3.84 (br. s, 4H), 2.09 (heptett, 4H).

### Beispiel 13

### 1-{2-[6-(4,4-Difluorpiperidin-1-yl)pyrimidin-4-yl]-3-oxo-2,3-dihydro-1H-pyrazol-yl}-1H-1,2,3-triazol-4-carbonitril

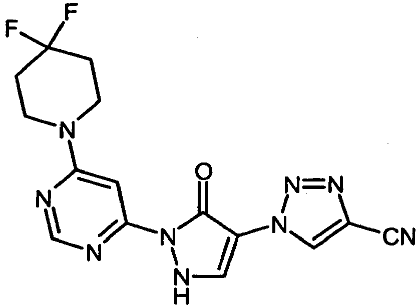

Ein Gemisch aus 200 mg (1.4 mmol) der Verbindung aus Beispiel 11A, 262 mg (1.7 mmol) 4,4-Difluorpiperidinhydrochlorid und 289 µl (215 mg, 1.7 mmol) *N*-Ethyl-*N*-(propan-2-yl)propan-2-amin in 3 ml Wasser wird 16 h bei 100°C gerührt. Nach Zugabe von 53 µl (79 mg, 0.7 mmol) Trifluoressigsäure und 325 mg (1.4 mmol) der Verbindung aus Beispiel 7A wird das Reaktionsgemisch 16 h bei 100°C gerührt. Der ausgefallene Feststoff wird abfiltriert und zunächst mit Wasser, dann mit Diethylether gewaschen. Das Produkt wird im Vakuum getrocknet.
Ausbeute: 34 mg (7% d. Th.)
LC-MS (Methode 4): Rₜ = 1.77 min; MS (ESIpos): m/z = 374 [M+H]⁺;
¹H-NMR (400 MHz, DMSO-d₆): δ = 9.24 (s, 1H), 8.59 (s, 1H), 8.27 (s, 1H), 7.54 (s, 1H), 3.90 (br. s, 4H), 2.12 (heptett, 4H).

### Beispiel 14

### 2-[6-(3,3-Difluorpyrrolidin-1-yl)pyrimidin-4-yl]-4-(1H-imidazol-1-yl)-1,2-dihydro-3H-pyrazol-3-on

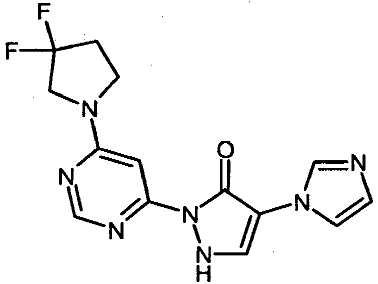

100 mg (0.3 mmol) der Verbindung aus Beispiel 13A, 58 mg (0.4 mmol) 3,3-Difluorpyrrolidin-hydrochlorid und 175 µl (130 mg, 1.0 mmol) *N*-Ethyl-*N*-(propan-2-yl)propan-2-amin werden in 2 ml Tetrahydrofuran vorgelegt und 1 h bei 120°C in einer *single mode*-Mikrowelle (Emrys Optimizer) umgesetzt. Nach Einengen im Vakuum wird der Rückstand in Acetonitril/Wasser aufgenommen und mittels präparativer HPLC (RP18-Säule; Laufmittel: Acetonitril/Wasser-Gradient) gereinigt. Ausbeute: 111 mg (99% d. Th.)
HPLC (Methode 6): Rₜ = 3.18 min; MS (ESIpos): m/z = 334 [M+H]⁺;
¹H-NMR (400 MHz, Methanol-d₄): δ = 8.41 (s, 1H), 7.85 (s, 1H), 7.62 (s, 1H), 7.56 (s, 1H), 7.29 (s, 1H), 7.03 (s, 1H), 3.91 (t, 2H), 3.76 (t, 2H), 2.55 (heptett, 2H).

### Beispiel 15

### 2-[6-(3,3-Difluorpyrrolidin-1-yl)pyrimidin-4-yl]-4-(1H-1,2,3-triazol-1-yl)-1,2-dihydro-3Hpyrazol-3-on

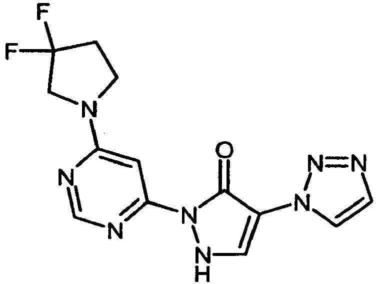

100 mg (0.8 mmol) der Verbindung aus Beispiel 12A, 57 mg (0.4 mmol) 3,3-Difluorpyrrolidinhydrochlorid und 174 µl (129 mg, 1.0 mmol) *N*-Ethyl-N-(propan-2-yl)propan-2-amin werden in 2 ml Tetrahydrofuran vorgelegt und 30 min bei 120°C in einer *single mode*-Mikrowelle (Emrys Optimizer) umgesetzt. Nach Einengen im Vakuum wird der Rückstand in Acetonitril/Wasser aufgenommen und mittels präparativer HPLC (RP18-Säule; Laufmittel: Acetonitril/Wasser-Gradient) gereinigt. Ausbeute: 13 mg (12% d. Th.)
HPLC (Methode 6): Rₜ = 3.33 min; MS (ESIpos): m/z = 335 [M+H]⁺;
¹H-NMR (400 MHz, DMSO-d₆): δ = 8.42 (s, 1H), 8.35 (s, 1H), 7.72-7.66 (m, 3H), 3.87 (t, 2H), 3.65 (t, 2H), 2.64-2.50 (m, teilweise unter dem DMSO-Signal, 2H).

### Beispiel 16

### 1-{2-[6-(3,3-Difluorpyrrolidin-1-yl)pyrimidin-4-yl]-3-oxo-2,3-dihydro-1H-pyrazol-4-yl}-1H-imidazol-4-carbonitril

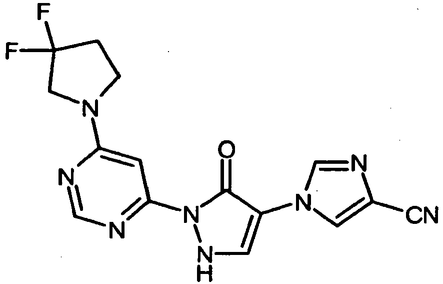

Ein Gemisch aus 200 mg (1.4 mmol) der Verbindung aus Beispiel 11A, 238 mg (1.7 mmol) 3,3-Difluorpyrrolidinhydrochlorid und 289 µl (215 mg, 1.7 mmol) *N*-Ethyl-*N*-(propan-2-yl)propan-2-amin in 3 ml Wasser wird 16 h bei 100°C gerührt. Nach Zugabe von 53 µl (79 mg, 0.7 mmol) Trifluoressigsäure und 324 mg (1.4 mmol) der Verbindung aus Beispiel 6A wird das Reaktionsgemisch 16 h bei 100°C gerührt. Das Reaktionsgemisch wird mit 1 ml 1 N Salzsäure versetzt. Das hierbei ausgefallene Hydrochlorid des Rohprodukts wird abfiltriert, mit Diethylether gewaschen und getrocknet. Das Rohprodukt enthält noch nicht umgesetzte Ausgangsverbindung (Verbindung aus Beispiel 11A). Daher wird das Rohprodukt mit 108 µl (80 mg, 0.6 mmol) *N-*Ethyl-*N*-(propan-2-yl)propan-2-amin, 10 mg (0.1 mmol) 3,3-Difluorpyrrolidinhydrochlorid und 2 ml Wasser 15 min bei 170°C in einer *single mode*-Mikrowelle (Emrys Optimizer) umgesetzt. Die Reaktionslösung wird mittels präparativer HPLC (RP18-Säule; Laufmittel: Acetonitril/Wasser-Gradient) gereinigt. Ausbeute: 30 mg (6% d. Th.)
LC-MS (Methode 4): Rₜ = 1.58 min; MS (ESIpos): m/z = 359 [M+H]⁺;
¹H-NMR (400 MHz, DMSO-d₆): δ = 8.55 (s, 1H), 8.44 (d, 1H), 8.33 (s, 1H), 8.21 (d, 1H), 7.26 (br. s, 1H), 3.99 (t, 2H), 3.75 (br. s, 2H), 2.66-2.54 (m, teilweise unter dem DMSO-Signal, 2H).

### Beispiel 17

### 1-{2-[6-(3,3-Difluorpyrrolidin-1-yl)pyrimidin-4-yl]-3-oxo-2,3-dihydro-1H-pyrazol-4-yl}-1H-1,2,3-triazol-4-carbonitril

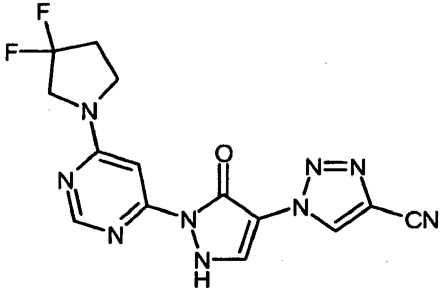

Ein Gemisch aus 200 mg (1.4 mmol) der Verbindung aus Beispiel 11A, 238 mg (1.7 mmol) 3,3-Difluorpyrrolidinhydrochlorid und 289 µl (215 mg, 1.7 mmol) *N*-Ethyl-*N*-(propan-2-yl)propan-2-amin in 3 ml Wasser wird 16 h bei 100°C gerührt. Nach Zugabe von 53 µl (79 mg, 0.7 mmol) Trifluoressigsäure und 325 mg (1.4 mmol) der Verbindung aus Beispiel 7A wird das Reaktionsgemisch 16 h bei 100°C gerührt. Der ausgefallene Feststoff wird abfiltriert und zunächst mit Wasser, dann mit Diethylether gewaschen. Das Produkt wird im Vakuum getrocknet.
Ausbeute: 137 mg (27% d. Th.)
LC-MS (Methode 4): Rₜ = 1.66 min; MS (ESIpos): m/z = 360 [M+H]⁺;
¹H-NMR (400 MHz, DMSO-d₆): δ = 9.25 (s, 1H), 8.61 (s, 1H), 8.29 (s, 1H), 7.21 (br. s, 1H), 4.06 (t, 2H), 3.82 (br. s, 2H), 2.69-2.55 (m, teilweise unter dem DMSO-Signal, 2H).

### Beispiel 18

### 2-[6-(3,3-Difluorazetidin-1-yl)pyrimidin-4-yl]-4-(1H-imidazol-1-yl)-1,2-dihydro-3H-pyrazol-3-on

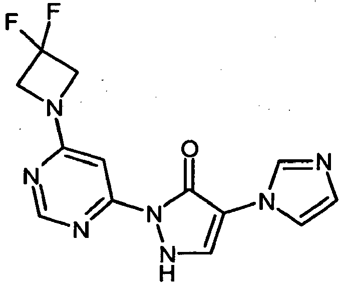

100 mg (0.3 mmol) der Verbindung aus Beispiel 13A, 52 mg (0.4 mmol) 3,3-Difluorazetidinhydrochlorid und 175 µl (130 mg, 1.0 mmol) *N*-Ethyl-*N*-(propan-2-yl)propan-2-amin werden in 2 ml Tetrahydrofuran vorgelegt und 3 h bei 120°C in einer *single mode*-Mikrowelle (Emrys Optimizer) umgesetzt. Der ausgefallene Feststoff wird abfiltriert und das Filtrat wird im Vakuum eingeengt. Der Rückstand wird in Acetonitril/Wasser aufgenommen und mittels präparativer HPLC (RP18-Säule; Laufmittel: Acetonitril/Wasser-Gradient) gereinigt. Ausbeute: 36 mg (32% d. Th.)
HPLC (Methode 6): Rₜ = 3.00 min; MS (ESIpos): m/z = 320 [M+H]⁺;
¹H-NMR (400 MHz, DMSO-4): δ = 8.42 (s, 1H), 8.16 (s, 1H), 7.79 (s, 1H), 7.56 (s, 1H), 7.50 (s, 1H), 7.07 (s, 1H), 4.49 (t, 4H).

### Beispiel 19

### 2-[6-(3,3-Difluorazetidin-1-yl)pyrimidin-4-yl]-4-(1H-1,2,3-triazol-1-yl)-1,2-dihydro-3H-pyrazol-3-on

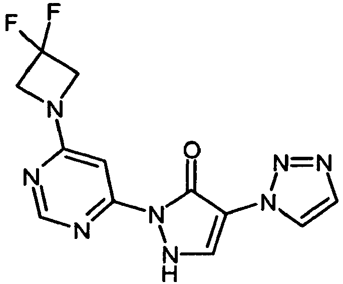

100 mg (0.3 mmol) der Verbindung aus Beispiel 12A, 52 mg (0.4 mmol) 3,3-Difluorazetidinhydrochlorid und 174 µl (130 mg, 1.0 mmol) *N*-Ethyl-*N*-(propan-2-yl)propan-2-amin werden in 2 ml Tetrahydrofuran vorgelegt und 30 min bei 120°C in einer *single mode*-Mikrowelle (Emrys Optimizer) umgesetzt. Nach Einengen im Vakuum wird der Rückstand in Acetonitril/Wasser aufgenommen und mittels präparativer HPLC (RP18-Säule; Laufmittel: Acetonitril/Wasser-Gradient) gereinigt. Ausbeute: 36 mg (34% d. Th.)
HPLC (Methode 6): Rₜ = 3.20 min; MS (ESIpos): m/z = 321 [M+H]⁺;
¹H-NMR (400 MHz, DMSO-d₆): δ = 8.42 (s, 1H), 8.39 (s, 1H), 7.71 (s, 2H), 7.62 (s, 1H), 4.46 (t, 4H).

### Beispiel 20

### 1-{2-[6-(3,3-Difluorazetidin-1-yl)pyrimidin-4-yl]-3-oxo-2,3-dihydro-1H-pyrazol-4-yl}-1H-imidazol-4-carbonitril

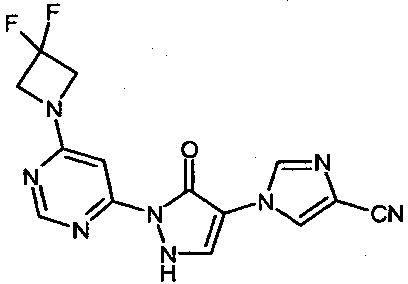

Ein Gemisch aus 120 mg (0.8 mmol) der Verbindung aus Beispiel 11A, 129 mg (1.0 mmol) 3,3-Difluorazetidinhydrochlorid und 174 µl (129 mg, 1.0 mmol) *N*-Ethyl-*N*-(propan-2-yl)propan-2-amin in 3 ml Wasser wird 16 h bei 100°C gerührt. Nach Zugabe von 32 µl (47 mg, 0.4 mmol) Trifluoressigsäure und 194 mg (0.8 mmol) der Verbindung aus Beispiel 6A wird das Reaktionsgemisch 16 h bei 100°C gerührt. Der ausgefallene Feststoff wird abfiltriert und zunächst mit Wasser, dann mit Diethylether gewaschen. Das Produkt wird im Vakuum getrocknet.
Ausbeute: 32 mg (11 % d. Th.)
LC-MS (Methode 4): Rₜ = 1.48 min; MS (ESIpos): m/z = 345 [M+H]⁺;
¹H-NMR (400 MHz, DMSO-d₆): δ = 8.80-8.08 (m, 4H), 7.25 (s, 1H), 4.61 (br. s, 4H).

### Beispiel 21

### 1-{2-[6-(3,3-Difluorazetidin-1-yl)pyrimidin-4-yl]-3-oxo-2,3-dihydro-1H-pyrazol-4-yl}-1H-1,2,3-triazol-4-carbonitril

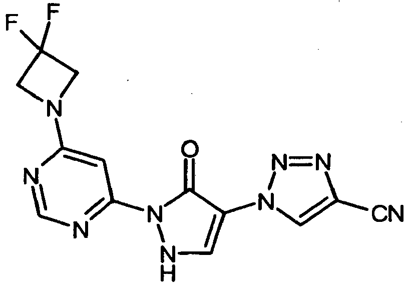

Ein Gemisch aus 120 mg (0.8 mmol) der Verbindung aus Beispiel 11A, 129 mg (1.0 mmol) 3,3-Difluorazetidinhydrochlorid und 174 µl (129 mg, 1.0 mmol) *N*-Ethyl-*N*-(propan-2-yl)propan-2-amin in 3 ml Wasser wird 16 h bei 100°C gerührt. Nach Zugabe von 32 µl (47 mg, 0.4 mmol) Trifluoressigsäure und 194 mg (0.8 mmol) der Verbindung aus Beispiel 7A wird das Reaktionsgemisch 16 h bei 100°C gerührt. Der ausgefallene Feststoff wird abfiltriert und zunächst mit Wasser, dann mit Diethylether gewaschen. Das Produkt wird im Vakuum getrocknet.
Ausbeute: 51 mg (18% d. Th.)
LC-MS (Methode 4): Rₜ = 1.56 min; MS (ESIpos): m/z = 346 [M+H]⁺;
¹H-NMR (400 MHz, DMSO-d₆): δ = 9.26 (s, 1H), 8.60 (s, 1H), 8.36 (s, 1H), 7.18 (s, 1H), 4.69 (t, 4H).

### Beispiel 22

### 4-(1H-Imidazol-1-yl)-2-[6-(1,4-oxazepan-4-yl)pyrimidin-4-yl]-1,2-dihydro-3H-pyrazol-3-on-Hydrochlorid

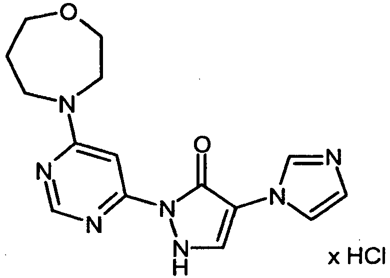

Ein Gemisch aus 200 mg (1.0 mmol) der Verbindung aus Beispiel 5A, 200 mg (1.0 mmol) der Verbindung aus Beispiel 14A und 37 µl (54 mg, 0.5 mmol) Trifluoressigsäure in 3 ml Wasser wird 16 h bei 100°C gerührt. Nach einer Vorreinigung mittels präparativer HPLC (RP18-Säule; Laufmittel: Acetonitril/Wasser-Gradient) wird das Rohprodukt mit 1 ml 1 N Salzsäure verrührt, abfiltriert und mit Diethylether gewaschen. Das Produkt wird im Vakuum getrocknet. Ausbeute: 21 mg (6% d. Th.)
LC-MS (Methode 4): Rₜ = 0.95 min; MS (ESIpos): m/z = 364 [M+H]⁺;
¹H-NMR (400 MHz, DMSO-d₆): δ = 9.46 (s, 1H), 8.56 (s, 1H), 8.30 (s, 1H), 8.06 (t, 1H), 7.83 (t, 1H), 7.50-7.33 (m, 1H), 4.05 (br. s, 2H), 3.91-3.70 (m, 4H), 3.68 (t, 2H), 2.04-1.73 (m, 2H).

### Beispiel 23

### 2-[6-(1,4-Oxazepan-4-yl)pyrimidin-4-yl]-4-(1H-1,2,3-triazol-1-yl)-1,2-dihydro-3H-pyrazol-3-on-Hydrochlorid

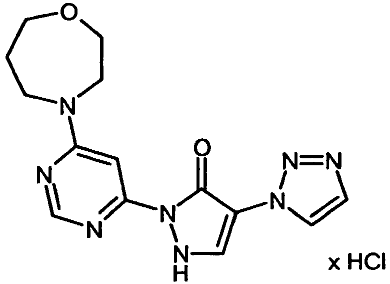

Ein Gemisch aus 400 mg (1.3 mmol) der Verbindung aus Beispiel 12A und 220 mg (1.6 mmol) 1,4-Oxazepanhydrochlorid werden in 4 ml Propan-2-ol 30 min bei 115°C in einer *single mode-*Mikrowelle (Emrys Optimizer) umgesetzt. 232 µl (172 mg, 1.3 mmol) *N*-Ethyl-*N*-(propan-2-yl)propan-2-amin werden addiert und das Reaktionsgemisch wird 20 min bei 115°C in einer *single mode*-Mikrowelle (Emrys Optimizer) umgesetzt. Nach Einengen im Vakuum wird der Rückstand in Acetonitril/Wasser/Trifluoressigsäure aufgenommen und mittels präparativer HPLC (Methode 9) chromatographiert. Das aus der HPLC-Trennung erhaltene, lyophilisierte Trifluoracetat-Salz wird mit 2 ml 1 N Salzsäure in das Hydrochlorid überführt. Ausbeute: 7 mg (1% d. Th.)
LC-MS (Methode 4): Rₜ = 1.20 min; MS (ESIpos): m/z = 329 [M+H]⁺;
¹H-NMR (400 MHz, DMSO-d₆): δ = 8.54 (s, 1H), 8.38 (s, 1H), 8.25 (s, 1H), 7.86 (d, 1H), 7.35 (br. s, 1H), 4.14-3.69 (m, 6H), 3.67 (t, 2H), 2.03-1.77 (m, 2H).

### Beispiel 24

### 2-[6-(1,4-Oxazepan-4-yl)pyrimidin-4-yl]-4-(1H-1,2,3-triazol-1-yl)-1,2-dihydro-3H-pyrazol-3-on

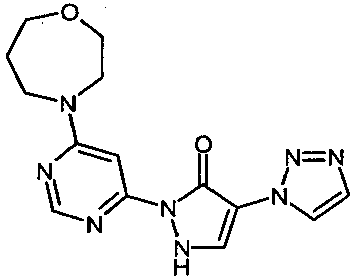

Ein Gemisch aus 500 mg (1.7 mmol) der Verbindung aus Beispiel 12A, 688 mg (5.0 mmol) 1,4-Oxazepanhydrochlorid und 1.4 ml (1077 mg, 8.3 mmol) *N*-Ethyl-*N*-(propan-2-yl)propan-2-amin werden in 10 ml Tetrahydrofuran/Ethanol (1/1) 30 min bei 140°C in einer *single mode*-Mikrowelle (Emrys Optimizer) umgesetzt. Nach einer Vorreinigung mittels präparativer HPLC (RP18-Säule; Laufmittel: Acetonitril/Wasser-Gradient) wird das Rohprodukt in Acetonitril/Trifluoressigsäure aufgenommen und mittels präparativer HPLC (Methode 10) chromatographiert. Das aus der HPLC-Trennung erhaltene, lyophilisierte Trifluoracetat-Salz wird mit 1 N Natronlauge auf pH = 7-8 gestellt. Das Produkt wird mittels präparativer HPLC (RP18-Säule; Laufmittel: Acetonitril/Wasser-Gradient) isoliert. Ausbeute: 176 mg (31% d. Th.)
LC-MS (Methode 4): Rₜ = 1.19 min; MS (ESIpos): m/z = 329 [M+H]⁺;
¹H-NMR (400 MHz, DMSO-d₆): δ = 8.41 (d, 1H), 8.33 (d, 1H), 7.78 (br. s, 1H), 7.72-7.70 (m, 2H), 3.91-3.65 (m, 6H), 3.62 (t, 2H), 1.95-1.83 (m, 2H).

### Beispiel 25

### 1-{2-[6-(1,4-Oxazepan-4-yl)pyrimidin-4-yl]-3-oxo-2,3-dihydro-1H-pyrazol-4-yl}-1H-imidazol-4-carbonitril

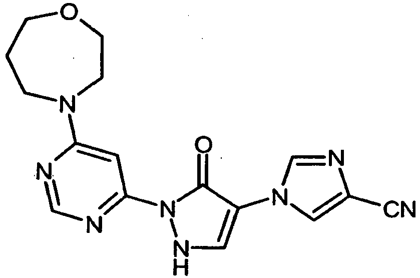

Ein Gemisch aus 200 mg (0.9 mmol) der Verbindung aus Beispiel 6A, 178 mg (0.9 mmol) der Verbindung aus Beispiel 14A und 33 µl (49 mg, 0.4 mmol) Trifluoressigsäure in 3 ml Wasser wird 16 h bei 100°C gerührt. Der ausgefallene Feststoff wird abfiltriert und zunächst mit Wasser, dann mit Diethylether gewaschen. Das Produkt wird im Vakuum getrocknet. Ausbeute: 120 mg (40% d. Th.)
HPLC (Methode 6): Rₜ = 3.27 min; MS (ESIpos): m/z = 353 [M+H]⁺;
¹H-NMR (400 MHz, DMSO-d₆): δ = 8.52 (s, 1H), 8.42 (d, 1H), 8.25 (s, 1H), 8.19 (d, 1H), 7.39 (br. s, 1H), 4.14-3.70 (m, 6H), 3.66 (t, 2H), 2.05-1.68 (m, 2H).

### Beispiel 26

### 1-{2-[6-(1,4-Oxazepan-4-yl)pyrimidin-4-yl]-3-oxo-2,3-dihydro-1H-pyrazol-4-yl}-1H-1,2,3-triazol-4-carbonitril

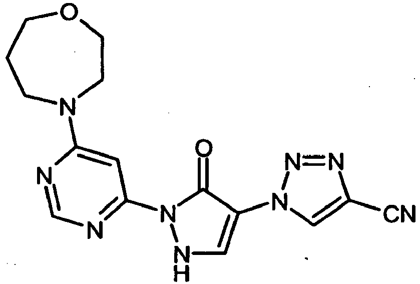

Ein Gemisch aus 200 mg (0.9 mmol) der Verbindung aus Beispiel 7A, 178 mg (0.9 mmol) der Verbindung aus Beispiel 14A und 33 µl (49 mg, 0.4 mmol) Trifluoressigsäure in 3 ml Wasser wird 16 h bei 100°C gerührt. Der ausgefallene Feststoff wird abfiltriert und zunächst mit Wasser, dann mit Diethylether gewaschen. Das Produkt wird im Vakuum getrocknet. Ausbeute: 80 mg (27% d. Th.)
LC-MS (Methode 4): Rₜ = 1.40 min; MS (ESIpos): m/z = 354 [M+H]⁺;
¹H-NMR (400 MHz, DMSO-d₆): δ = 9.22 (s, 1H), 8.56 (s, 1H), 8.21 (s, 1H), 7.45-7.26 (m, 1H), 4.06 (br. s, 2H), 3.92-3.71 (m, 4H), 3.68 (t, 2H), 2.06-1.74 (m, 2H).

### Beispiel 27

### 2-[6-(1,2-Oxazinan-2-yl)pyrimidin-4-yl]-4-(1H-1,2,3-triazol-1-yl)-1,2-dihydro-3H-pyrazol-3-on

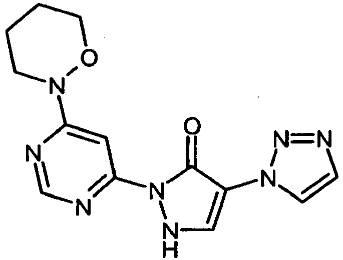

Ein Gemisch aus 200 mg (0.7 mmol) der Verbindung aus Beispiel 12A, 99 mg (0.8 mmol) 1,2-Oxazinanhydrochlorid [Bhat et al., J. Chem. Soc. Perkin Trans. 2 2000, 7, 1435-1446] und 348 µl (258 mg, 2.0 mmol) *N-*Ethyl-*N-*(propan-2-yl)propan-2-amin werden in 4 ml Tetrahydrofuran 30 min bei 140°C in einer *single mode*-Mikrowelle (Emrys Optimizer) umgesetzt. Nach einer Vorreinigung mittels präparativer HPLC (RP18-Säule; Laufmittel: Acetonitril/Wasser-Gradient) wird das Rohprodukt in Acetonitril/Wasser/Methanol aufgenommen und mittels präparativer HPLC (Methode 11) chromatographiert. Das aus der HPLC-Trennung erhaltene, lyophilisierte Formiat-Salz wird mit 1 N Natronlauge auf pH = 7-8 gestellt. Das Produkt wird mittels präparativer HPLC (RP18-Säule; Laufmittel: Acetonitril/Wasser-Gradient) isoliert. Ausbeute: 23 mg (11% d. Th.)
LC-MS (Methode 4): Rₜ = 1.38 min; MS (ESIpos): m/z = 315 [M+H]⁺;
¹H-NMR (400 MHz, DMSO-d₆): δ = 8.58 (d, 1H), 8.42 (d, 1H), 8.40 (br. s, 1H), 7.88 (d, 1H), 7.66 (br. s, 1H), 4.09 (t, 2H), 3.98 (t, 2H), 1.86-1.68 (m, 4H).

### Beispiel 28

### 1-{2-[6-(1,2-Oxazinan-2-yl)pyrimidin-4-yl]-3-oxo-2,3-dihydro-1H-pyrazol-4-yl}-1H-imidazol-4-carbonitril

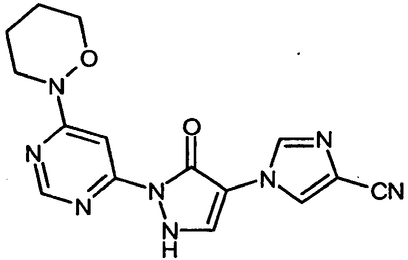

Ein Gemisch aus 200 mg (1.4 mmol) der Verbindung aus Beispiel 11A, 205 mg (1.7 mmol) 1,2-Oxazinanhydrochlorid [Bhat et al., J. Chem. Soc. Perkin Trans. 2 2000, 7, 1435-1446] und 289 µl (215 mg, 1.7 mmol) *N*-Ethyl-*N-*(propan-2-yl)propan-2-amin in 3 ml Wasser wird 1.5 h bei 100°C gerührt. Nach Zugabe von 59 µl (49 mg, 0.4 mmol) Trifluoressigsäure und 324 mg (1.4 mmol) der Verbindung aus Beispiel 6A wird das Reaktionsgemisch 16 h bei 100°C gerührt. Der ausgefallene Feststoff wird abfiltriert und zunächst mit Wasser, dann mit Diethylether gewaschen. Das Produkt wird im Vakuum getrocknet. Ausbeute: 199 mg (39% d. Th.)
LC-MS (Methode 5): Rₜ = 0.87 min; MS (ESIpos): m/z = 339 [M+H]⁺;
¹H-NMR (400 MHz, DMSO-d₆): δ = 8.57 (d, 1H), 8.45 (d, 1H), 8.40 (br. s, 1H), 8.23 (d, 1H), 7.70 (br. s, 1H), 4.07 (t, 2H), 3.96 (t, 2H), 1.85-1.65 (m, 4H).

### Beispiel 29

### 1-{2-[6-(1,2-Oxazinan-2-yl)pyrimidin-4-yl]-3-oxo-2,3-dihydro-1Hpyrazol-4-yl}-1H-1,2,3-triazol-4-carbonitril

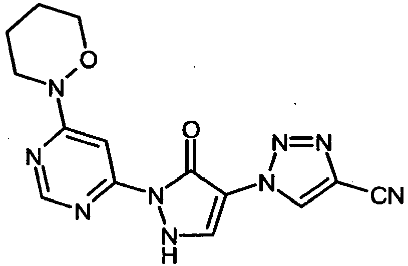

Ein Gemisch aus 200 mg (1.4 mmol) der Verbindung aus Beispiel 11A, 205 mg (1.7 mmol) 1,2-Oxazinanhydrochlorid [Bhat et al., J. Chem. Soc. Perkin Trans. 2 2000, 7, 1435-1446] und 289 µl (215 mg, 1.7 mmol) *N*-Ethyl-*N*-(propan-2-yl)propan-2-amin in 3 ml Wasser wird 1.5 h bei 100°C gerührt. Nach Zugabe von 59 µl (49 mg, 0.4 mmol) Trifluoressigsäure und 325 mg (1.4 mmol) der Verbindung aus Beispiel 7A wird das Reaktionsgemisch 16 h bei 100°C gerührt. Der ausgefallene Feststoff wird abfiltriert und zunächst mit Wasser, dann mit Diethylether gewaschen. Das Produkt wird im Vakuum getrocknet. Ausbeute: 122 mg (24% d. Th.)
LC-MS (Methode 4): Rₜ = 1.66 min; MS (ESIpos): m/z = 340 [M+H]⁺;
¹H-NMR (400 MHz, DMSO-d₆): δ = 9.28 (s, 1H), 8.58 (s, 1H), 8.36 (s, 1H), 7.60 (br. s, 1H), 4.12 (t, 2H), 4.03 (t, 2H), 1.88-1.70 (m, 4H).

### B. Bewertung der pharmakologischen Wirksamkeit

Die pharmakologischen Eigenschaften der erfindungsgemäßen Verbindungen können in folgenden Assays gezeigt werden:

### Abkürzungen:

- DMEM: Dulbecco's Modified Eagle Medium
- FCS: Fetal Calf Serum
- TMB: 3,3',5,5'-Tetramethylbenzidin
- Tris: Tris(hydroxymethyl)-aminomethan

### 1. In vitro-Tests zur Bestimmung der Aktivität und Selektivität von HIF-Prolyl-4-Hydroxylase-Inhibitoren

### 1.a) Hemmung der Aktivität von HIF-Prolylhydroxylase:

Hydroxylierter HIF bindet spezifisch an den von Hippel-Lindau Protein-Elongin B-Elongin C-Komplex (VBC-Komplex). Diese Interaktion tritt nur auf, wenn HIF an einem konservierten Prolylrest hydroxyliert ist. Sie ist die Grundlage für die biochemische Bestimmung der HIF-Prolylhydroxylase-Aktivität. Der Test wird wie beschrieben durchgeführt [Oehme F., Jonghaus W., Narouz-Ott L., Huetter J., Flamme I., Anal. Biochem. 330 (1), 74-80 (2004)]:

Eine klare, mit NeutrAvidin HBC beschichtete 96-Loch-Mikrotiterplatte (Fa. Pierce) wird für 30 Minuten mit Blocker-Casein inkubiert. Anschließend wird die Platte dreimal mit je 200 µl Waschpuffer (50 mM Tris, pH 7.5, 100 mM NaCl, 10% (v/v) Blocker-Casein, 0.05% (v/v) Tween 20) pro Loch gewaschen. Das Peptid Biotin-DLDLEMLAPYIPMDDDFQL (Fa. Eurogentec, 4102 Seraing, Belgien) wird in einer Konzentration von 400 nM in 100 µl Waschpuffer zugegeben. Dieses Peptid dient als Substrat für die Prolylhydroxylierung und wird an die Mikrotiterplatte gebunden. Nach 60 Minuten Inkubation wird die Platte dreimal mit Waschpuffer gewaschen, 30 Minuten mit 1 mM Biotin in Blocker-Casein inkubiert und dann erneut dreimal mit Waschpuffer gewaschen.

Zur Durchführung der Prolylhydroxylase-Reaktion wird das an die Platte gebundene Peptidsubstrat 1 bis 60 Minuten mit einem Prolylhydroxylase-haltigen Zelllysat inkubiert. Die Reaktion findet in 100 µl Reaktionspuffer (20 mM Tris, pH 7.5, 5 mM KCI, 1.5 mM MgCl₂, 1 µM-1 mM 2-Oxoglutarat, 10 µM FeSO₄, 2 mM Ascorbat) bei Raumtemperatur statt. Die Reaktionsmischung enthält außerdem den zu testenden Hemmstoff der Prolylhydroxylase in verschiedenen Konzentrationen.

Die Testsubstanz wird bevorzugt, aber nicht ausschließlich bei Konzentrationen zwischen 1 nM und 100 µM eingesetzt. Durch dreimaliges Waschen der Platte mit Waschpuffer wird die Reaktion gestoppt.

Zur quantitativen Bestimmung der Prolylhydroxylierung wird ein Fusionsprotein, das sowohl Thioredoxin aus E. coli als auch den VBC-Komplex enthält, in 80 µl Bindungspuffer (50 mM Tris, pH 7.5, 120 mM NaCl) zugegeben. Nach 15 Minuten werden 10 µl einer Lösung von polyklonalem Anti-Thioredoxin-Antikörper aus Kaninchen in Bindungspuffer zugefügt. Nach weiteren 30 Minuten setzt man 10 µl einer Lösung von mit Meerrettich-Peroxidase gekoppeltem Anti-Kaninchen-Immunglobulin in Bindungspuffer hinzu. Nach 30 Minuten Inkubation bei Raumtemperatur wird dreimal mit Waschpuffer gewaschen, um ungebundenen VBC-Komplex und Antikörper zu entfernen. Um die Menge an gebundenem VBC-Komplex zu bestimmen, wird 15 Minuten mit TMB inkubiert. Die Farbreaktion wird durch Zugabe von 100 µl 1 M Schwefelsäure beendet. Durch Messung der optischen Dichte bei 450 nm wird die Menge an gebundenem VBC-Komplex bestimmt. Sie ist proportional zur Menge an hydroxyliertem Prolin im Peptidsubstrat.

Zur Detektion der Prolylhydroxylierung kann alternativ ein mit Europium (Fa. Perkin Elmer) gekoppelter VBC-Komplex verwendet werden. In diesem Fall wird die Menge an gebundenem VBC-Komplex durch zeitaufgelöste Fluoreszenz bestimmt. Außerdem ist die Verwendung von mit [³⁵S]-Methionin markiertem VBC-Komplex möglich. Hierfür kann der radioaktiv markierte VBC-Komplex durch *in vitro-*Transkription-Translation in Retikulozytenlysat hergestellt werden.

Die Ausführungsbeispiele hemmen die Aktivität der HIF-Prolylhydroxylase in diesem Test mit einem IC₅₀-Wert von ≤ 30 µM. In der nachstehenden Tabelle 1 sind repräsentative IC₅₀-Werte zu den Ausführungsbeispielen wiedergegeben:

**Tabelle 1**

| **Beispiel Nr.** | **IC₅₀ [nM]** | | **Beispiel Nr.** | **IC₅₀ [nM]** |
|---|---|---|---|---|
| **2** | 880 | | **21** | 70 |
| **5** | 540 | | **25** | 180 |
| **9** | 760 | | **26** | 380 |
| **17** | 130 | | **29** | 170 |
| **20** | 90 | | | |

### 1.b) Zellulärer, funktioneller in vitro-Test:

Die Quantifizierung der Wirksamkeit der erfindungsgemäßen Verbindungen erfolgt mit Hilfe einer rekombinanten Zelllinie. Die Zelle leitet sich ursprünglich von einer humanen Lungencarcinom-Zelllinie ab (A549, ATCC: American Type Culture Collection, Manassas, VA 20108, USA). Die Testzelllinie wird stabil mit einem Vektor transfiziert, der das Reportergen der *Photinus pyralis-*Luciferase (im folgenden Luciferase genannt) unter der Kontrolle eines artifiziellen Minimalpromotors enthält. Der Minimalpromotor besteht aus zwei Hypoxie-responsiblen Elementen stromaufwärts einer TATA-Box [Oehme F., Ellinghaus P., Kolkhof P., Smith T.J., Ramakrishnan S., Hütter J., Schramm M., Flamme I., Biochem. Biophys. Res. Commun. 296 (2), 343-9 (2002)]. Unter Einwirkung von Hypoxie (z.B. Kultivierung in Gegenwart von 1% Sauerstoff für 24 Stunden) oder unter Einwirkung unselektiver Dioxygenase-Inhibitoren (z.B. Desferroxamin in einer Konzentration von 100 µM, Cobaltchlorid in einer Konzentration von 100 µM oder *N*-Oxalylglycindiethylester in einer Konzentration von 1 mM) produziert die Testzelllinie Luciferase, die mit Hilfe geeigneter Biolumineszenz-Reagenzien (z.B. Steady-Glo^{®} Luciferase Assay System, Promega Corporation, Madison, WI 53711, USA) und eines geeigneten Luminometers detektiert und quantifiziert werden kann.

Testablauf: Die Zellen werden am Tag vor dem Test in einer exakt bemessenen Menge Kulturmedium (DMEM, 10% FCS, 2 mM Glutamin) in 384- oder 1536-Loch-Mikrotiterplatten ausplattiert und in einem Zellinkubator (96% Luftfeuchtigkeit, 5% v/v CO₂, 37°C) gehalten. Am Testtag werden dem Kulturmedium die Testsubstanzen in abgestuften Konzentrationen zugesetzt. In als Negativkontrolle dienenden Ansätzen wird den Zellen keine Testsubstanz zugesetzt. Als Positivkontrolle zur Bestimmung der Empfindlichkeit der Zelle für Inhibitoren wird z.B. Desferroxamin in einer Endkonzentration von 100 µM zugesetzt. Sechs bis 24 Stunden nach Übertragung der Testsubstanzen in die Löcher der Mikrotiterplatten wird das resultierende Lichtsignal im Luminometer gemessen. Anhand der Meßwerte wird eine Dosiswirkungsbeziehung aufgestellt, die als Grundlage für die Ermittlung der halbmaximalen Wirkkonzentration (als EC₅₀-Wert bezeichnet) dient.

### 1.c) Zellulärer, funktioneller in vitro-Test zur Veränderung der Genexpression:

Um die Veränderung der Expression spezifischer mRNAs in menschlichen Zelllinien nach Behandlung mit Testsubstanzen zu untersuchen, werden folgende Zelllinien auf 6- oder 24-Lochplatten kultiviert: humane Hepatomzellen (HUH, JCRB Cell Bank, Japan), humane embryonale Nierenfibroblasten (HEK/293, ATCC, Manassas, VA 20108, USA), humane Cervixcarcinomzellen (HeLa, ATCC, Manassas, VA 20108, USA), humane Nabelschnurvenenendothelzellen (HUVEC, Cambrex, East Rutherford, New Jersey 07073, USA). 24 Stunden nach Zugabe der Testsubstanzen werden die Zellen mit Phosphat-gepufferter Saline gewaschen und aus ihnen die Gesamt-RNA unter Verwendung einer geeigneten Methode gewonnen (z.B. Trizol^{®}-Reagenz, Invitrogen GmbH, 76131 Karlsruhe, Deutschland).

Für ein typisches Analyseexperiment wird je 1 µg der so gewonnenen Gesamt-RNA mit DNase I verdaut und unter Verwendung einer geeigneten Reversen-Transkriptase-Reaktion (ImProm-II Reverse Transcription System, Promega Corporation, Madison, WI 53711, USA) in eine komplementäre DNA (cDNA) übersetzt. 2.5% des so gewonnenen cDNA-Ansatzes werden jeweils für die Polymerase-Kettenreaktion verwendet. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht. Die hierbei benutzten Primer-Sonden-Kombinationen werden mittels der Primer Express 1.5 Software (Fa. Applied Biosystems, Inc.) generiert. Im einzelnen werden die mRNAs von Erythropoetin, Carboanhydrase IX, Lactatdehydrogenase A und vaskulärem Endothelzellwachstumsfaktor untersucht.

Substanzen gemäß der vorliegenden Erfindung führen zu einem signifikanten, dosisabhängigen Anstieg der mRNA Hypoxie-induzierter Gene in Zellen menschlichen Ursprungs.

### 2. In vivo-Tests zum Nachweis der Wirkung im Kardiovaskularsystem

### 2.a) In vivo-Test zur Veränderung der Genexpression:

Mäusen oder Ratten werden die in geeigneten Lösungsmitteln gelösten Prüfverbindungen entweder oral durch Schlundsonden-Applikation, intraperitoneal oder intravenös verabfolgt. Typische Dosierungen sind 0.1, 0.5, 1, 5, 10, 20, 50, 100 und 300 mg Substanz pro kg Körpergewicht und Verabfolgung. Kontrolltiere erhalten nur Lösungsmittel. 4, 8 oder 24 Stunden nach Gabe der Prüfsubstanz werden die Tiere mit einer Überdosis Isofluran und anschließendem Genickbruch getötet und die zu untersuchenden Organe entnommen. Teile der Organe werden in flüssigem Stickstoff schockgefroren. Aus den Organteilen wird wie unter B.1.a) beschrieben Gesamt-RNA gewonnen und diese in eine cDNA übersetzt. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht.

Substanzen gemäß der vorliegenden Erfindung führen im Vergleich mit der Placebo-Kontrolle nach oraler oder parenteraler Verabreichung zu einem signifikanten, dosisabhängigen Anstieg der mRNA des Erythropoetins in der Niere.

### 2.b) Bestimmung des Erythropoetin-Spiegels im Serum:

Mäusen oder Ratten wird die Prüfsubstanz in einem geeigneten Lösungsmittel entweder intraperitoneal oder oral einmal oder zweimal täglich verabreicht. Typische Dosierungen sind 0.1, 0.5, 1, 5, 10, 20, 50, 100 und 300 mg Substanz pro kg Körpergewicht und Verabfolgung. Placebo-Kontrolltiere erhalten nur Lösungsmittel. Vor der Applikation und vier Stunden nach der letzten Substanzgabe wird den Tieren in Kurznarkose aus dem retroorbitalen Venenplexus oder der Schwanzvene 50 µl Blut entnommen. Das Blut wird durch Zusatz von Lithium-Heparin ungerinnbar gemacht. Durch Zentrifugieren wird das Blutplasma gewonnen. In dem Blutplasma wird mit Hilfe eines Erythropoetin-ELISA (Quantikine^{®} mouse Epo Immunoassay, R&D Systems, Inc., Minneapolis, USA) entsprechend der Anleitung des Herstellers der Gehalt an Erythropoetin bestimmt. Die Meßwerte werden anhand einer für Maus-Erythropoetin erhobenen Referenzmessung in pg/ml umgerechnet.

Substanzen gemäß der vorliegenden Erfindung führen nach oraler und parenteraler Verabreichung zu einem signifikanten, dosisabhängigen Anstieg des Plasma-Erythropoetins gegenüber dem Ausgangswert und der Placebo-Kontrolle.

### 2.c) Bestimmung der zellulären Zusammensetzung des peripheren Blutes:

Mäusen oder Ratten wird die Prüfsubstanz in einem geeigneten Lösungsmittel entweder intraperitoneal oder oral einmal oder zweimal täglich über mehrere Tage verabreicht. Typische Dosierungen sind z.B. 0.1, 0.5, 1, 5, 10, 20, 50, 100 und 300 mg Substanz pro kg Körpergewicht und Verabfolgung. Kontrolltiere erhalten nur Lösungsmittel. Am Versuchsende wird den Tieren in Kurznarkose aus dem Venenplexus des Augenwinkels oder der Schwanzvene Blut entnommen und durch Zusatz von Natriumcitrat ungerinnbar gemacht. In einem geeigneten elektronischen Messgerät werden in den Blutproben die Konzentrationen von Erythrozyten, Leukozyten und Thrombozyten bestimmt. Die Konzentration der Retikulozyten wird anhand von Blutausstrichen, die mit einer dafür geeigneten Farblösung (Fa. KABE Labortechnik, Nümbrecht) gefärbt werden, durch mikroskopische Durchmusterung von je 1000 Erythrozyten bestimmt. Für die Bestimmung des Hämatokrits wird Blut aus dem retroorbitalen Venenplexus mittels einer Hämatokritkapillare entnommen und der Hämatokritwert nach Zentrifugieren der Kapillare in einer dafür geeigneten Zentrifuge manuell abgelesen.

Substanzen gemäß der vorliegenden Erfindung führen nach oraler und parenteraler Verabreichung zu einem signifikanten, dosisabhängigen Anstieg des Hämatokrits, der Erythrozytenzahl und der Retikulozyten gegenüber dem Ausgangswert und der Placebo-Kontrolle.

### 3. Bestimmung der Löslichkeit

### Herstellung der Ausgangslösung (Urlösung):

Mindestens 1.5 mg der Testsubstanz werden in ein Wide Mouth 10 mm Screw V-Vial (Fa. Glastechnik Gräfenroda GmbH, Art.-Nr. 8004-WM-H/V15µ) mit passender Schraubkappe und Septum genau eingewogen, mit DMSO zu einer Konzentration von 50 mg/ml versetzt und 30 Minuten mittels eines Vortexers geschüttelt.

### Herstellung der Kalibrierlösungen:

Die notwendigen Pipettierschritte erfolgen in 1.2 ml 96er Deep Well Plate (DWP) mittels eines Liquid-Handling-Roboters. Als Lösemittel wird ein Gemisch aus Acetonitril/Wasser 8:2 verwendet.

*Herstellung der Ausgangslösung für Kalibrierlösungen (Stammlösung):* 10 µl der Urlösung werden mit 833 µl des Lösemittelgemisch versetzt (Konzentration = 600 µg/ml) und homogenisiert. Es werden von jeder Testsubstanz 1:100 Verdünnungen in separaten DWP's hergestellt und wiederum homogenisiert.

*Kalibrierlösung 5 (600 ng*/*ml):* 30 µl der Stammlösung werden mit 270 µl Lösemittelgemisch versetzt und homogenisiert.

*Kalibrierlösung 4 (60 ng*/*ml):* 30 µl der Kalibrierlösung 5 werden mit 270 µl Lösemittelgemisch versetzt und homogenisiert.

*Kalibrierlösung 3 (12 ng*/*ml):* 100 µl der Kalibrierlösung 4 werden mit 400 µl Lösemittelgemisch versetzt und homogenisiert.

*Kalibrierlösung 2 (1.2 ng*/*ml):* 30 µl der Kalibrierlösung 3 werden mit 270 µl Lösemittelgemisch versetzt und homogenisiert.

*Kalibrierlösung 1 (0.6 ng*/*ml):* 150 µl der Kalibrierlösung 2 werden mit 150 µl Lösemittelgemisch versetzt und homogenisiert.

### Herstellung der Probenlösungen:

Die notwendigen Pipettierschritte erfolgen in 1.2 ml 96er DWP mittels eines Liquid-Handling-Roboters. 10.1 µl der Stammlösung werden mit 1000 µl PBS-Puffer pH 6.5 versetzt. (PBS-Puffer pH 6.5: 61.86 g Natriumchlorid, 39.54 g Natriumdihydrogenphosphat und 83.35 g 1 N Natronlauge werden in einen 1 Liter-Messkolben eingewogen, mit Wasser aufgefüllt und ca. 1 Stunde gerührt. Von dieser Lösung werden 500 ml in einen 5 Liter-Messkolben gegeben und mit Wasser aufgefüllt. Es wird mit 1 N Natronlauge auf pH 6.5 einstellen.)

### Durchführung:

Die notwendigen Pipettierschritte erfolgen in 1.2 ml 96er DWP mittels eines Liquid-Handling-Roboters. Die so hergestellten Probenlösungen werden 24 Stunden bei 1400 rpm mittels eines temperierbaren Schüttlers bei 20°C geschüttelt. Von diesen Lösungen werden jeweils 180 µl abgenommen und in Beckman Polyallomer Centrifuge Tubes überführt. Diese Lösungen werden 1 Stunde mit ca. 223.000 x g zentrifugiert. Von jeder Probenlösung werden 100 µl des Überstandes abgenommen und 1:10 und 1:1000 mit PBS-Puffer 6.5 verdünnt.

### Analytik:

Die Proben werden mittels HPLC/MS-MS analysiert. Quantifiziert wird über eine Fünf-Punkt-Kalibrationskurve der Testverbindung. Die Löslichkeit wird in mg/l ausgedrückt. Analysensequenz: 1) Blank (Lösemittelgemisch); 2) Kalibrierlösung 0.6 ng/ml; 3) Kalibrierlösung 1.2 ng/ml; 4) Kalibrierlösung 12 ng/ml; 5) Kalibrierlösung 60 ng/ml; 6) Kalibrierlösung 600 ng/ml; 7) Blank (Lösemittelgemisch); 8) Probenlösung 1:1000; 7) Probenlösung 1:10.

### HPLC/MS-MSMethode

HPLC: Agilent 1100, quat. Pumpe (G1311A), Autosampler CTC HTS PAL, Degaser (G1322A) und Säulenthermostat (G1316A); Säule: Oasis HLB 20 mm x 2.1 mm, 25 µ; Temperatur: 40°C; Eluent A: Wasser + 0.5 ml Ameisensäure/l; Eluent B: Acetonitril + 0.5 ml Ameisensäure/l; Flussrate: 2.5 ml/min; Stoptime 1.5 min; Gradient: 0 min 95% A, 5% B; Rampe: 0-0.5 min 5% A, 95% B; 0.5-0.84 min 5% A, 95% B; Rampe: 0.84-0.85 min 95% A, 5% B; 0.85-1.5 min 95% A, 5% B.

MS/MS: WATERS Quattro Micro Tandem MS/MS; Z-Spray API-Interface; HPLC-MS-Eingangssplitter 1:20; Messung im ESI-Mode.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel 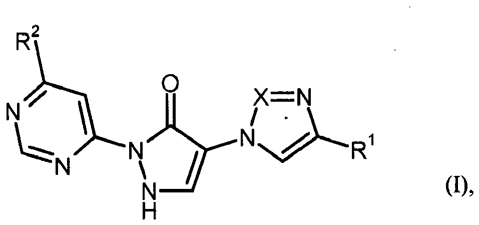 in welcher
X für N oder CH steht,
R¹ für Wasserstoff oder Cyano steht,
R² für Piperazin-1-yl steht,
wobei Piperazin-1-yl substituiert ist mit einem Substituenten, wobei der Substituent ausgewählt wird aus der Gruppe bestehend aus C₃-C₆-Cycloalkyl,
oder eines ihrer Salze, ihrer Solvate oder der Solvate ihrer Salze.

2. Verbindung nach Anspruch 1, **dadurch gekennzeichnet, dass**
X für N oder CH steht,
R¹ für Wasserstoff oder Cyano steht,
R² für Piperazin-1-yl steht,
wobei Piperazin-1-yl in 4-Position substituiert ist mit einem Substituenten, wobei der Substituent ausgewählt wird aus der Gruppe bestehend aus C₃-C₆-Cycloalkyl,
oder eines ihrer Salze, ihrer Solvate oder der Solvate ihrer Salze.

3. Verbindung nach Anspruch 1 oder 2, **dadurch gekennzeichnet, dass**
X für N oder CH steht,
R¹ für Wasserstoff steht,
R² für Piperazin-1-yl steht,
wobei Piperazin-1-yl in 4-Position substituiert ist mit einem Substituenten, wobei der Substituent ausgewählt wird aus der Gruppe bestehend aus C₃-C₆-Cycloalkyl,
oder eines ihrer Salze, ihrer Solvate oder der Solvate ihrer Salze.

4. Verbindung nach Anspruch 1 oder 2, **dadurch gekennzeichnet, dass**
X für N oder CH steht,
R¹ für Wasserstoff oder Cyano steht,
R² für 4-Cyclobutyl-piperazin-1-yl steht,
oder eines ihrer Salze, ihrer Solvate oder der Solvate ihrer Salze.

5. 2-[6-(4-Cyclobutylpiperazin-1-yl)pyrimidin-4-yl]-4-(1*H*-1,2,3-triazol-1-yl)-1,2-dihydro-3H-pyrazol-3-on nach Anspruch 1 mit der folgenden Formel 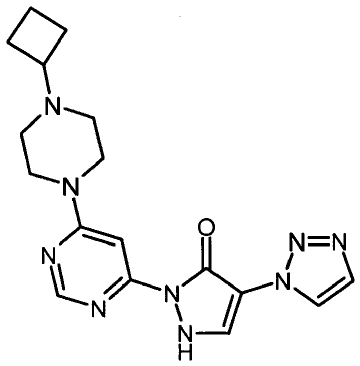 oder eines ihrer Salze, ihrer Solvate oder der Solvate ihrer Salze.

6. 2-[6-(4-Cyclobutylpiperazin-1-yl)pyrimidin-4-yl]-4-(1*H*-1,2,3-triazol-1-yl)-1,2-dihydro-3*H* pyrazol-3-on nach Anspruch 5 mit der folgenden Formel

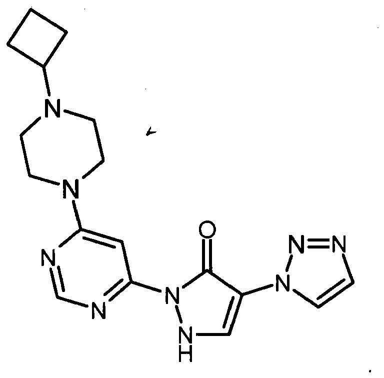

7. Verfahren zur Herstellung einer Verbindung der Formel (I) oder eines ihrer Salze, ihrer Solvate oder der Solvate ihrer Salze nach Anspruch 1, **dadurch gekennzeichnet, dass**
[A] eine Verbindung der Formel 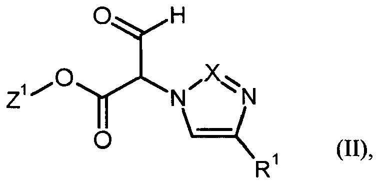 in welcher R¹ die in Anspruch 1 angegebene Bedeutung hat, und
Z¹ für Methyl oder Ethyl steht,
in einem inerten Lösungsmittel gegebenenfalls in Gegenwart einer Säure mit einer Verbindung der Formel 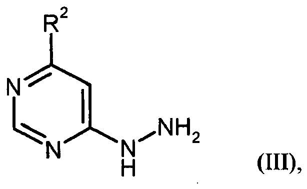 in welcher R² die in Anspruch 1 angegebene Bedeutung hat,
zu einer Verbindung der Formel 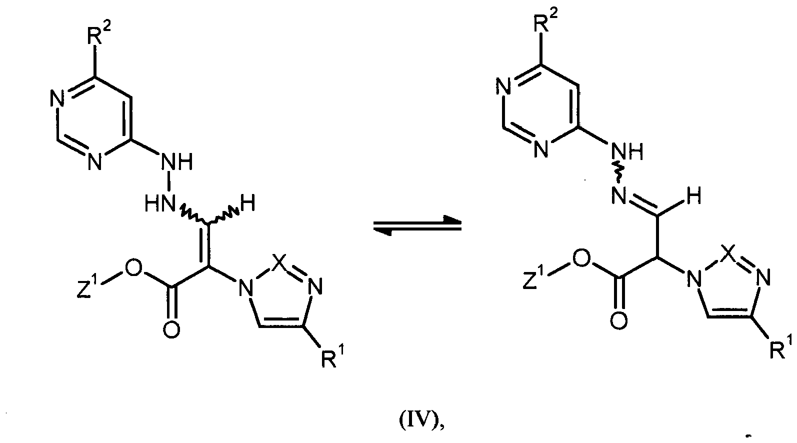 in welcher Z¹, R¹ und R² die in Anspruch 1 angegebene Bedeutung haben,
umgesetzt wird, welche bereits unter diesen Reaktionsbedingungen oder in einem nachfolgenden Reaktionsschritt unter dem Einfluss einer Base zu der Verbindung der Formel (I) cyclisiert,
und die Verbindung der Formel (I) gegebenenfalls mit dem entsprechenden (i) Lösungsmittel und/oder (ii) Base oder Säure in eines ihrer Salze, ihrer Solvate oder der Solvate ihrer Salze überführt wird,
oder
[B] eine Verbindung der Formel 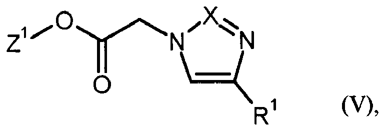 in welcher Z¹ und R¹ die in Anspruch 1 angegebene Bedeutung haben,
mit einer Verbindung der Formel 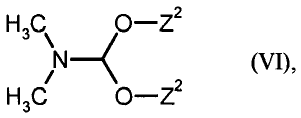 in welcher
Z² für Methyl oder Ethyl steht,
zu einer Verbindung der Formel 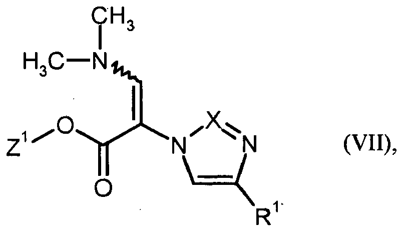 in welcher Z¹ und R¹ die in Anspruch 1 angegebene Bedeutung haben,
kondensiert wird und anschließend in Gegenwart einer Säure mit einer Verbindung der Formel (III) zu einer Verbindung der Formel (IV) umgesetzt wird, welche bereits unter diesen Reaktionsbedingungen oder in einem nachfolgenden Reaktionsschritt unter dem Einfluss einer Base zu der Verbindung der Formel (I) cyclisiert,
und die Verbindung der Formel (I) gegebenenfalls mit dem entsprechenden (i) Lösungsmittel und/oder (ii) Base oder Säure in eines ihrer Salze, ihrer Solvate oder der Solvate ihrer Salze überführt wird,
oder
[C] die Verbindung der Formel 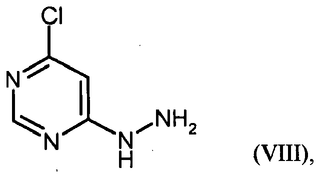 in Wasser als Lösungsmittel in einem Eintopfverfahren zunächst mit einer Verbindung der Formel 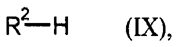 in welcher R² die in Anspruch 1 angegebene Bedeutung hat,
und anschließend mit einer Verbindung der Formel (VII) zu einer Verbindung der Formel (I) umgesetzt wird,
und die Verbindung der Formel (I) gegebenenfalls mit dem entsprechenden (i) Lösungsmittel und/oder (ii) Base oder Säure in eines ihrer Salze, ihrer Solvate oder der Solvate ihrer Salze überführt wird.

8. Verbindung nach einem der Ansprüche 1 bis 6 zur Behandlung und/oder Prophylaxe von Krankheiten.

9. Verwendung einer Verbindung nach einem der Ansprüche 1 bis 6 zur Herstellung eines Arzneimittels zur Behandlung und/oder Prophylaxe von Krankheiten.

10. Verwendung einer Verbindung nach einem der Ansprüche 1 bis 6 zur Herstellung eines Arzneimittels zur Behandlung und/oder Prophylaxe von Herz-Kreislauf-Erkrankungen, Herzinsuffizienz, Anämie, chronischen Nierenerkrankungen und Niereninsuffizienz.

11. Arzneimittel enthaltend eine Verbindung nach einem der Ansprüche 1 bis 6 in Kombination mit einem inerten, nichttoxischen, pharmazeutisch geeigneten Hilfsstoff.

12. Arzneimittel nach Anspruch 11, in Kombination mit einem oder mehreren weiteren Wirkstoffen ausgewählt aus der Gruppe bestehend aus ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika und Antibiotika.

13. Arzneimittel nach Anspruch 11 oder 12 zur Behandlung und/oder Prophylaxe von Herz-Kreislauf-Erkrankungen, Herzinsuffizienz, Anämie, chronischen Nierenerkrankungen und Niereninsuffizienz.

14. Verbindung, wie in einem der Ansprüche 1 bis 6 definiert, zur Verwendung in einem Verfahren zur Behandlung und/oder Prophylaxe von Herz-Kreislauf-Erkrankungen, Herzinsuffizienz, Anämie, chronischen Nierenerkrankungen und Niereninsuffizienz.

## Claims

1. Compound of the formula 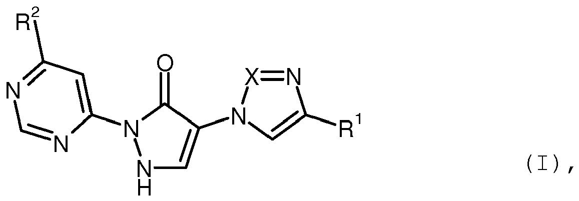 in which
X represents N or CH,
R¹ represents hydrogen or cyano,
R² represents piperazin-1-yl,
where piperazin-1-yl is substituted by a substituent, where the substituent is selected from the group consisting of C₃-C₆-cycloalkyl,
or one of the salts, solvates or solvates of the salts thereof.

2. Compound according to Claim 1, **characterized in that**
X represents N or CH,
R¹ represents hydrogen or cyano,
R² represents piperazin-1-yl,
where piperazin-1-yl is substituted in the 4-position by one substituent, where the substituent is selected from the group consisting of C₃-C₆-cycloalkyl,
or one of the salts, solvates or solvates of the salts thereof.

3. Compound according to Claim 1 or 2, **characterized in that**
X represents N or CH,
R¹ represents hydrogen,
R² represents piperazin-1-yl,
where piperazin-1-yl is substituted in the 4-position by one substituent, where the substituent is selected from the group consisting of C₃-C₆-cycloalkyl,
or one of the salts, solvates or solvates of the salts thereof.

4. Compound according to Claim 1 or 2, **characterized in that**
X represents N or CH,
R¹ represents hydrogen or cyano,
R² represents 4-cyclobutylpiperazin-1-yl,
or one of the salts, solvates or solvates of the salts thereof.

5. 2-[6-(4-Cyclobutylpiperazin-1-yl)pyrimidin-4-yl]-4-(1*H*-1,2,3-triazol-1-yl)-1,2-dihydro-3H-pyrazol-3-one according to Claim 1 having the following formula 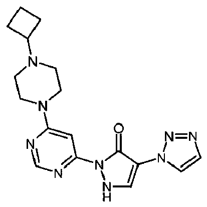 or one of the salts, solvates or solvates of the salts thereof.

6. 2-[6-(4-Cyclobutylpiperazin-1-yl)pyrimidin-4-yl]-4-(1*H*-1,2,3-triazol-1-yl)-1,2-dihydro-3*H*-pyrazol-3-one according to Claim 5 having the following formula

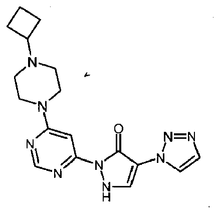

7. Process for preparing a compound of the formula (I) or a salt, solvate or solvate of the salt thereof according to Claim 1, **characterized in that**
[A] a compound of the formula 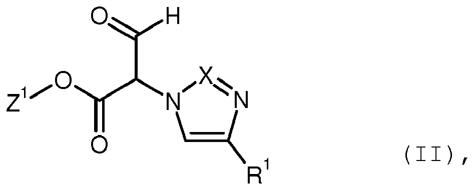 in which R¹ has the meaning given in Claim 1, and
Z¹ represents methyl or ethyl,
is reacted in an inert solvent, if appropriate in the presence of an acid, with a compound of the formula 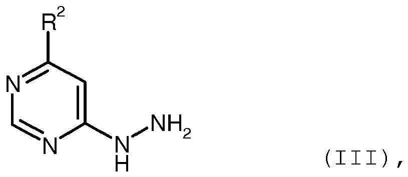 in which R² has the meaning given in Claim 1, to give a compound of the formula 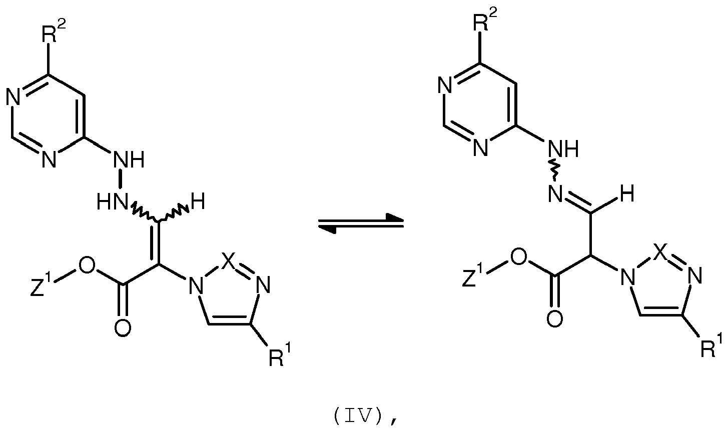 in which Z¹, R¹ and R² have the meaning given in Claim 1,
which, already under these reaction conditions or in a subsequent reaction step under the influence of a base, cyclizes to give the compound of the formula (I),
and the compound of the formula (I) is, if appropriate with the appropriate (i) solvent and/or (ii) base or acid, converted into one of its salts, its solvates or solvates of its salts, or
[B] a compound of the formula 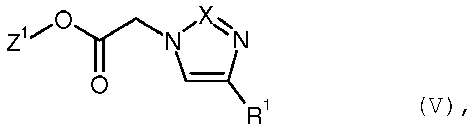 in which Z¹ and R¹ have the meaning given in Claim 1,
is condensed with a compound of the formula 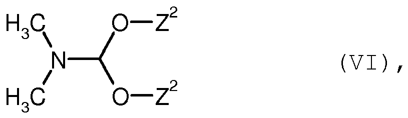 in which
Z² represents methyl or ethyl,
to give a compound of the formula 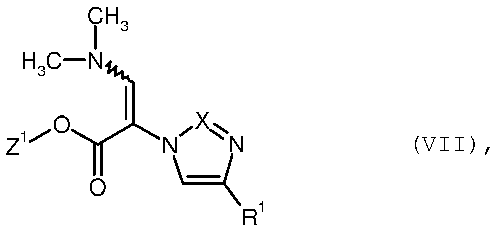 in which Z¹ and R¹ have the meaning given in Claim 1,
and then reacted in the presence of an acid with a compound of the formula (III) to give a compound of the formula (IV), which, already under these reaction conditions or in a subsequent reaction step under the influence of a base, cyclizes to give the compound of the formula (I),
and the compound of the formula (I) is, if appropriate with the appropriate (i) solvent and/or (ii) base or acid, converted into one of its salts, its solvates or solvates of its salts,
or
[C] the compound of the formula 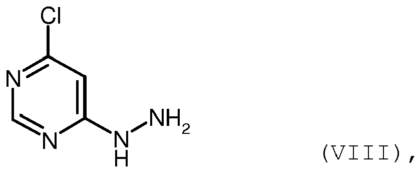 is, in water as the solvent in a one-pot process, reacted initially with a compound of the formula 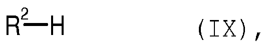 in which R² has the meaning given in Claim 1,
and then with a compound of the formula (VII) to give a compound of the formula (I),
and the compound of the formula (I) is, if appropriate with the appropriate (i) solvent and/or (ii) base or acid, converted into one of its salts, its solvates or solvates of its salts.

8. Compound according to any of Claims 1 to 6 for treatment and/or prophylaxis of diseases.

9. Use of a compound according to any of Claims 1 to 6 for the preparation of a medicament for treatment and/or prophylaxis of diseases.

10. Use of a compound according to any of Claims 1 to 6 for the preparation of a medicament for treatment and/or prophylaxis of cardiovascular diseases, cardiac insufficiency, anaemia, chronic kidney diseases and renal insufficiency.

11. Medicament comprising a compound according to any of Claims 1 to 6 in combination with an inert, non-toxic, pharmaceutically suitable auxiliary.

12. Medicament according to Claim 11, in combination with one or more further active compounds chosen from the group consisting of ACE inhibitors, angiotensin II receptor antagonists, beta receptor blockers, calcium antagonists, PDE inhibitors, mineralocorticoid receptor antagonists, diuretics, aspirin, iron supplements, vitamin B12 and folic acid supplements, statins, digitalis (digoxin) derivatives, tumour chemotherapeutics and antibiotics.

13. Medicament according to Claim 11 or 12 for treatment and/or prophylaxis of cardiovascular diseases, cardiac insufficiency, anaemia, chronic kidney diseases and renal insufficiency.

14. Compound as defined in any of Claims 1 to 6 for use in a method for treatment and/or prophylaxis of cardiovascular diseases, cardiac insufficiency, anaemia, chronic kidney diseases and renal insufficiency.

## Revendications

1. Composé de formule 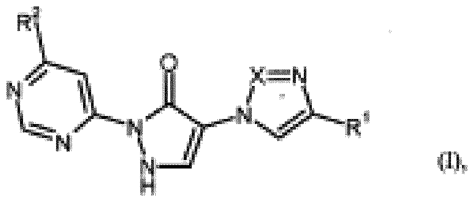 dans laquelle
X représente N ou CH,
R¹ représente un atome d'hydrogène ou le groupe cyano,
R² représente un groupe pipérazin-1-yle,
le groupe pipérazin-1-yle étant substitué par un substituant, le substituant étant choisi dans le groupe constitué par cycloalkyle en C₃-C₆,
ou l'un de ses sels, de ses produits de solvatation ou des produits de solvatation de ses sels.

2. Composé selon la revendication 1, **caractérisé en ce que**
X représente N ou CH,
R¹ représente un atome d'hydrogène ou le groupe cyano,
R² représente un groupe pipérazin-1-yle,
le groupe pipérazin-1-yle étant substitué en position 4 par un substituant, le substituant étant choisi dans le groupe constitué par cycloalkyle en C₃-C₆,
ou l'un de ses sels, de ses produits de solvatation ou des produits de solvatation de ses sels.

3. Composé selon la revendication 1 ou 2, **caractérisé en ce que**
X représente N ou CH,
R¹ représente un atome d'hydrogène,
R² représente un groupe pipérazin-1-yle,
le groupe pipérazin-1-yle étant substitué en position 4 par un substituant, le substituant étant choisi dans le groupe constitué par cycloalkyle en C₃-C₆,
ou l'un de ses sels, de ses produits de solvatation ou des produits de solvatation de ses sels.

4. Composé selon la revendication 1 ou 2, **caractérisé en ce que**
X représente N ou CH,
R¹ représente un atome d'hydrogène ou le groupe cyano,
R² représente le groupe 4-cyclobutyl-pipérazin-l-yle,
ou l'un de ses sels, de ses produits de solvatation ou des produits de solvatation de ses sels.

5. 2-[6-(4-cyclobutylpipérazin-1-yl)pyrimidin-4-yl]-4-(1*H*-1,2,3-triazol-1-yl)-1,2-dihydro-3*H*-pyrazol-3-one selon la revendication 1, ayant la formule suivante 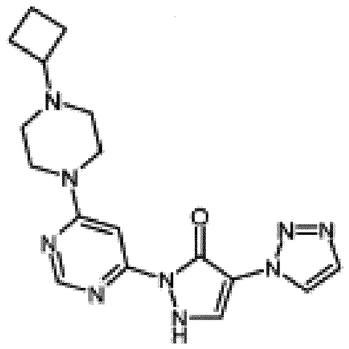 ou l'un de ses sels, de ses produits de solvatation ou des produits de solvatation de ses sels.

6. 2-[6-(4-cyclobutylpipérazin-1-yl)pyrimidin-4-yl]-4-(1*H*-1,2,3-triazol-1-yl)-1,2-dihydro-3*H*-pyrazol-3-one selon la revendication 5, ayant la formule suivante

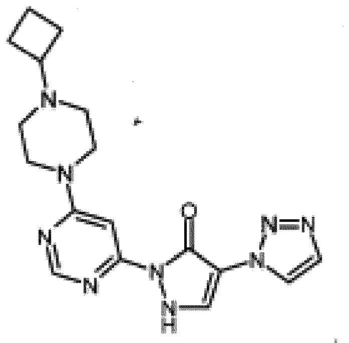

7. Procédé pour la préparation d'un composé de formule (I) ou d'un de ses sels, de ses produits de solvatation ou des produits de solvatation de ses sels selon la revendication 1, **caractérisé en ce que**
[A] on fait réagir un composé de formule 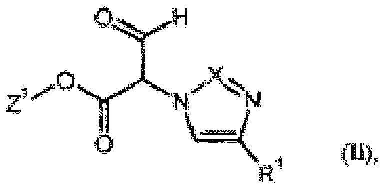 dans laquelle R¹ a la signification indiquée dans la revendication 1, et
Z¹ représente le groupe méthyle ou éthyle,
dans un solvant inerte éventuellement en présence d'un acide, avec un composé de formule 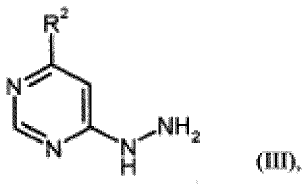 dans laquelle R² a la signification indiquée dans la revendication 1,
pour obtenir un composé de formule 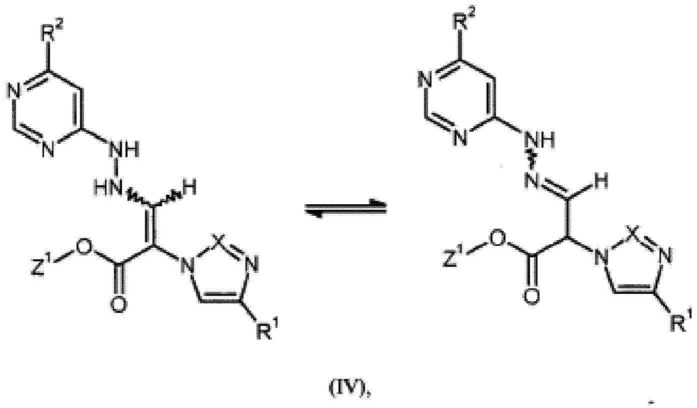 dans laquelle Z¹, R¹ et R² ont la signification indiquée dans la revendication 1,
qui, déjà dans ces conditions réactionnelles ou dans une étape de réaction subséquente, cyclise sous l'effet d'une base, en le composé de formule (I),
et éventuellement on convertit le composé de formule (I), à l'aide (i) du solvant correspondant et/ou (ii) de la base correspondante ou de l'acide correspondant, en l'un de ses sels, de ses produits de solvatation ou des produits de solvatation de ses sels
ou
[B] on condense un composé de formule 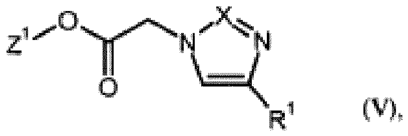 dans laquelle Z¹ et R¹ ont la signification indiquée dans la revendication 1,
avec un composé de formule 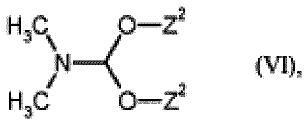 dans laquelle
Z² représente le groupe méthyle ou éthyle,
pour aboutir à un composé de formule 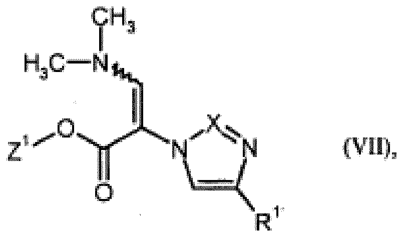 dans laquelle Z¹ et R¹ ont la signification indiquée dans la revendication 1,
et ensuite on le fait réagir en présence d'un acide avec un composé de formule (III) pour obtenir un composé de formule (IV) qui, déjà dans ces conditions réactionnelles ou dans une étape de réaction subséquente, cyclise sous l'effet d'une base, en le composé de formule (I),
et éventuellement on convertit le composé de formule (I), à l'aide (i) du solvant correspondant et/ou (ii) de la base correspondante ou de l'acide correspondant, en l'un de ses sels, de ses produits de solvatation ou des produits de solvatation de ses sels,
ou
[C] on fait réagir le composé de formule 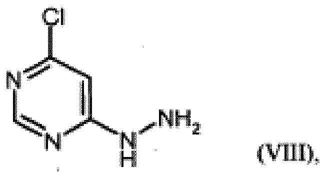 dans de l'eau en tant que solvant, dans un procédé en un seul récipient, d'abord avec un composé de formule 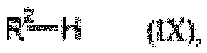 dans laquelle R² a la signification indiquée dans la revendication 1,
et ensuite avec un composé de formule (VII), pour obtenir un composé de formule (I),
et éventuellement on convertit le composé de formule (I), à l'aide (i) du solvant correspondant et/ou (ii) de la base correspondante ou de l'acide correspondant, en l'un de ses sels, de ses produits de solvatation ou des produits de solvatation de ses sels.

8. Composé selon l'une quelconque des revendications 1 à 6, destiné au traitement et/ou à la prophylaxie de maladies.

9. Utilisation d'un composé selon l'une quelconque des revendications 1 à 6, pour la fabrication d'un médicament destiné au traitement et/ou à la prophylaxie de maladies.

10. Utilisation d'un composé selon l'une quelconque des revendications 1 à 6, pour la fabrication d'un médicament destiné au traitement et/ou à la prophylaxie d'affections cardiovasculaires, d'insuffisance cardiaque, d'anémie, de néphropathies chroniques et d'insuffisance rénale.

11. Médicament contenant un composé selon l'une quelconque des revendications 1 à 6, en association avec un adjuvant inerte, non toxique, pharmaceutiquement approprié.

12. Médicament selon la revendication 11, en association avec une ou plusieurs autres substances actives choisies dans le groupe constitué par les inhibiteurs d'ACE, antagonistes des récepteurs d'angiotensine II, bêtabloquants, antagonistes du calcium, Inhibiteurs de PDE, antagonistes des récepteurs de minéralocorticoïdes, diurétiques, l'aspirine, les suppléments de fer, les suppléments de vitamine B12 et d'acide folique, les statines, les dérivés de digitale (digoxine), les agents chimiothérapeutiques-antitumoraux et les antibiotiques.

13. Médicament selon la revendication 11 ou 12, destiné au traitement et/ou à la prophylaxie d'affections cardiovasculaires, d'insuffisance cardiaque, d'anémie, de néphropathies chroniques et d'insuffisance rénale.

14. Composé, tel que défini dans l'une quelconque des revendications 1 à 6, destiné à l'utilisation dans un procédé pour le traitement et/ou la prophylaxie d'affections cardiovasculaires, d'insuffisance cardiaque, d'anémie, de néphropathies chroniques et d'insuffisance rénale.
